(19) Europäisches Patentamt
European Patent Office
Office européen des brevets

(11) Veröffentlichungsnummer: **0 438 690 B1**

(12) # EUROPÄISCHE PATENTSCHRIFT

(45) Veröffentlichungstag der Patentschrift: **06.07.94**

(51) Int. Cl.$^{5}$: **C07D 403/04**, A01N 43/48, A01N 43/713, A01N 43/64

(21) Anmeldenummer: **90123775.0**

(22) Anmeldetag: **11.12.90**

Die Akte enthält technische Angaben, die nach dem Eingang der Anmeldung eingereicht wurden und die nicht in dieser Patentschrift enthalten sind.

(54) **Substituierte Pyrazolinderivate.**

(30) Priorität: **24.01.90 DE 4001931**
**10.10.90 DE 4032089**

(43) Veröffentlichungstag der Anmeldung:
**31.07.91 Patentblatt 91/31**

(45) Bekanntmachung des Hinweises auf die Patenterteilung:
**06.07.94 Patentblatt 94/27**

(84) Benannte Vertragsstaaten:
**BE CH DE FR GB IT LI NL**

(56) Entgegenhaltungen:
**EP-A- 0 167 028**
**DE-A- 2 529 689**
**DE-A- 2 700 258**

(73) Patentinhaber: **BAYER AG**

**D-51368 Leverkusen(DE)**

(72) Erfinder: **Fuchs, Rainer, Dr.**
**Am Rohm 107**
**W-5600 Wuppertal 1(DE)**
Erfinder: **Wachendorff-Neumann, Ulrike, Dr.**
**Kriescherstrasse 81**
**W-4019 Monheim(DE)**
Erfinder: **Becker, Benedikt, Dr.**
**Via Max Sparer 17**
**I-39057 Appiano (BZ)(IT)**
Erfinder: **Erdelen, Christoph, Dr.**
**Unterbüscherhof 22**
**W-5653 Leichlingen 1(DE)**
Erfinder: **Stendel, Wilhelm, Dr.**
**In den Birken 55**
**W-5600 Wuppertal 1(DE)**

EP 0 438 690 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue substituierte Pyrazolinderivate, Verfahren zu ihrer Herstellung und ihre Verwendung als Schädlingsbekämpfungsmittel.

Es ist bekannt, daß bestimmte substituierte Pyrazolinderivate eine gute Wirksamkeit gegen tierische Schädlinge besitzen.

Siehe dazu z.B. DE-A 2 700 258, US-A 4 174 393, DE-A 2 529 689, US-A 4 070 365.

Die Wirkungshöhe bzw. Wirkungsdauer dieser vorbekannten Verbindungen ist jedoch, insbesondere gegen bestimmte Organismen oder bei niedrigen Anwendungskonzentrationen, nicht in allen Anwendungsgebieten völlig zufriedenstellend.

Es wurden neue substituierte Pyrazolinderivate der allgemeinen Formel (I)

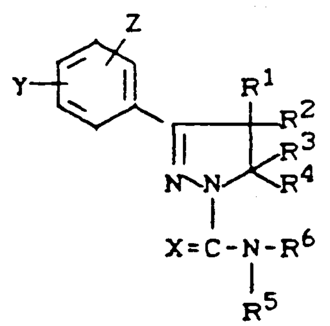

(I)

in welcher

$R^1$ für einen gegebenenfalls durch Halogen, Alkyl($C_1$-$C_6$), CN, $NO_2$, Alkoxy($C_1$-$C_6$)carbonyl, Alkyl($C_1$-$C_4$) thio, Alkoxy($C_1$-$C_6$), Halogenalkyl($C_1$-$C_4$), Halogenalkyl($C_1$-$C_4$)thio, Halogenalkoxy ($C_1$-$C_4$), Dialkyl($C_1$-$C_4$)amino oder Dihalogenamino substituierten 1H-Pyrrolyl-(1)-, 1H-Pyrazolyl-(1)-, 1H-Imidazolyl-(1)-, 2H-1,2,3,-Triazolyl-(2)-, 1H-1,2,3-Triazolyl-(1)-, 1H-1,2,4,-Triazolyl-(1)-, 4H-1,2,4-Triazolyl-(4)-, 2H-Tetrazolyl-(2)- oder 1H-Tetrazolyl-(2)-Rest steht,

$R^2$ für Wasserstoff, Alkyl($C_1$-$C_6$), gegebenenfalls durch Halogen, Halogenalkyl($C_1$-$C_4$) substituiertes Cycloalkyl($C_3$-$C_7$); Halogenalkyl($C_1$-$C_4$)thio, Alkoxy ($C_1$-$C_6$)carbonyl oder Trialkyl($C_1$-$C_6$)silyl steht.

$R^3$ für Wasserstoff oder Alkyl ($C_1$-$C_6$) steht,

$R^4$ für Wasserstoff oder Alkyl($C_1$-$C_6$) steht,

$R^5$ für Wasserstoff, Alkyl($C_1$-$C_6$), Phenyl oder Alkyl ($C_1$-$C_4$)thio steht,

$R^6$ für gegebenenfalls durch Halogen, Halogenalkyl($C_1$-$C_4$), Halogenalkoxy($C_1$-$C_4$) substituiertes Alkyl($C_1$-$C_6$),
für gegebenenfalls durch Halogen, Halogenalkyl($C_1$-$C_4$) Halogenalkoxy($C_1$-$C_4$) substituiertes Cycloalkyl ($C_3$-$C_7$) oder für den Rest

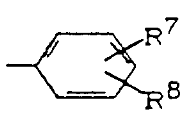

steht,
wobei $R^7$ und $R^8$ gleich oder verschieden sein können und für Wasserstoff, Halogen, Alkyl($C_1$-$C_6$), Nitro, Cyano, Halogenalkyl($C_1$-$C_6$), Alkoxy($C_1$-$C_6$), Halogenalkoxy ($C_1$-$C_6$), Alkyl($C_1$-$C_6$)thio, Halogenalkyl($C_1$-$C_6$)thio, gegebenenfalls durch Halogen, Halogenalkyl($C_1$-$C_6$), Alkoxy($C_1$-$C_6$), Alkyl($C_1$-$C_6$) substituiertes Phenoxy oder Phenylthio, gegebenenfalls durch Halogen, Alkoxy($C_1$-$C_4$), Halogenalkyl($C_1$-$C_4$) substituiertes Mono- oder Dialkylamino mit jeweils 1 bis 6 Kohlenstoffatomen im Alkylrest, gegebenenfalls durch Alkyl ($C_1$-$C_4$), Alkoxy($C_1$-$C_4$), Halogen, Alkyl($C_1$-$C_4$)thio substituiertes Cycloalkyl($C_3$-$C_7$), Alkoxy ($C_1$-$C_6$)carbonyl, Alkenyl($C_2$-$C_6$)oxy, Alkinyl($C_2$-$C_6$), Alkyl-($C_1$-$C_4$)thionyl, Alkyl($C_1$-$C_4$)sulfonyl, Halogenalkyl($C_1$-$C_4$)thionyl, Halogenalkyl($C_1$-$C_4$)sulfonyl, Halogenalkoxy($C_1$-$C_4$)-carbonyl stehen oder wobei
$R^7$ und $R^8$ zusammen für einen der folgenden bivalenten Reste stehen:

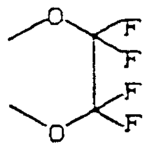 , 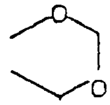 , 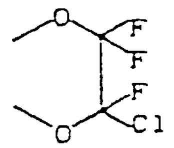 , 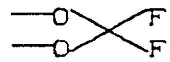 ,

X für Sauerstoff oder Schwefel steht und

Y und Z gleich oder verschieden sein können und für Wasserstoff, Alkyl($C_1$-$C_6$), Halogen, Halogenalkyl($C_1$-$C_6$), Alkoxy($C_1$-$C_6$), Alkyl($C_1$-$C_6$)thio, Halogenalkoxy($C_1$-$C_4$), Halogenalkyl($C_1$-$C_4$)-thio, Alkoxy($C_1$-$C_4$)carbonyl, Halogenalkoxy($C_1$-$C_4$)-carbonyl, gegebenenfalls durch Halogen, Alkyl($C_1$-$C_4$), Alkoxy($C_1$-$C_4$), Halogenalkyl ($C_1$-$C_4$) substituiertes Phenoxy oder Phenylthio, Alkenyl($C_2$-$C_6$)oxy, Alkinyl($C_2$-$C_6$), Alkyl($C_1$-$C_4$)-thionyl, Alkyl($C_1$-$C_4$)sulfonyl, Halogenalkyl($C_1$-$C_4$)-thionyl, Halogenalkyl($C_1$-$C_4$)sulfonyl, gegebenenfalls durch Halogen, Alkoxy($C_1$-$C_4$), Halogenalkyl-($C_1$-$C_4$) substituiertes Mono-, oder Dialkyl($C_1$-$C_6$)amino, Nitro, Cyano stehen oder wobei

Y und Z zusammen für gegebenenfalls durch Fluor und/oder Chlor substituiertes 3,4-Methylendioxy oder 3,4-Ethylendioxy stehen,

gefunden.

Weiterhin wurde gefunden, daß man die neuen substituierten Pyrazolinderivate der allgemeinen Formel (I)

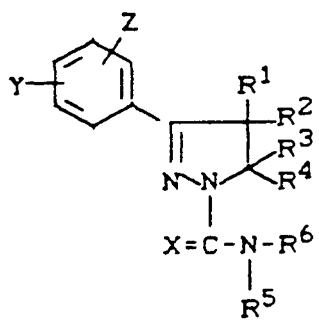 (I)

in welcher

$R^1$, $R^2$, $R^3$, $R^4$, $R^5$, $R^6$, X, Y und Z die oben angegebene Bedeutung haben,

erhält, wenn man

(a) zum Erhalt von Pyrazolinderivaten der Formel (I), in welcher $R^5$ für Wasserstoff steht, Pyrazolinderivate der Formel (II)

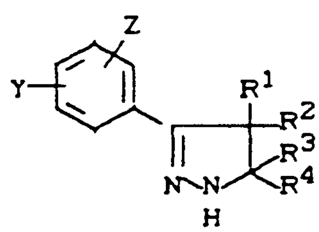 (II)

in welcher Y, Z, $R^1$, $R^2$, $R^3$ und $R^4$ die oben angegebene Bedeutung besitzen,

mit Isocyanaten bzw. Isothiocyanaten der Formel (III)

$X = C = N\text{-}R^6$ (III)

in welcher X und $R^6$ die oben angegebene Bedeutung haben,

gegebenenfalls in Gegenwart von Basen umsetzt

oder wenn man

(b) zum Erhalt von Pyrazolinderivaten der Formel (I), in welcher $R^2$ für Alkyl, Cycloalkyl, Halogenalkyl, Halogenalkylthio, Alkoxycarbonyl oder Trialkylsilyl steht, Verbindungen der Formel (IV)

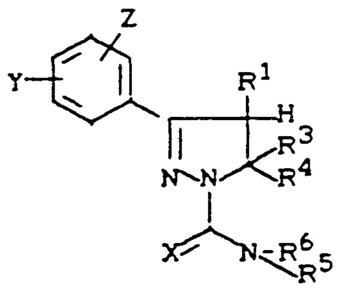

(IV)

mit Verbindungen der Formel (V)

Hal-$R^9$ (V)

in welcher

Hal für Halogen steht und

$R^9$ für Alkyl, Cycloalkyl, Halogenalkyl, Halogenalkylthio oder Alkoxycarbonyl steht, in wasserfreiem Medium unter Zusatz einer starken Base umsetzt.

Schließlich wurde gefunden, daß die neuen Pyrazolinderivate der allgemeinen Formel (I) eine sehr gute Wirksamkeit gegen Schädlinge und insbesondere eine sehr gute insektizide und akarizide Wirksamkeit besitzen.

Überraschenderweise zeigen die erfindungsgemäßen substituierten Pyrazolinderivate eine erheblich bessere insektizide Wirksamkeit gegen pflanzenschädigende und warmblüterparasitierende Insekten und Spinnentiere als aus dem Stand der Technik bekannte chemisch und wirkungsmäßig naheliegende Verbindungen.

Die erfindungsgemäßen substituierten Pyrazolinderivate sind durch die Formel (I) allgemein definiert. Bevorzugt sind Verbindungen der Formel (I), bei welchen

$R^1$ für einen gegebenenfalls durch Fluor, Chlor, Brom, Jod, Alkyl($C_1$-$C_4$), CN, $NO_2$, Alkoxy($C_1$-$C_4$)-carbonyl, Alkyl($C_1$-$C_3$)thio, Alkoxy($C_1$-$C_4$), Halogenalkyl ($C_1$-$C_3$), Halogenalkyl($C_1$-$C_3$)thio oder Halogenalkoxy($C_1$-$C_4$)carbonyl substituierten 1-H-Pyrrolyl-(1)-, 1H-Pyrazolyl-(1)-, 1H-Imidazolyl-(1)-, 2H-1,2,3-Triazolyl-(2)-, 1H-1,2,3-Triazolyl-(1)-, 1H,1,2,4-Triazolyl-(1)-, 4H-1,2,4-Triazolyl-(4)-, 2H-Tetrazolyl-(2) oder 1H-Tetrazolyl-(1)-Rest steht,

$R^2$ für Wasserstoff, Alkyl($C_1$-$C_4$), gegebenenfalls durch Fluor, Chlor, Brom, Halogenalkyl($C_1$-$C_3$)-substituiertesCycloalkyl ($C_3$-$C_6$), Halogenalkyl($C_1$-$C_3$), Halogenalkyl($C_1$-$C_3$)thio, Alkoxy($C_1$-$C_4$)-carbonyl steht oder Trialkyl($C_1$-$C_4$)silyl steht.

$R^3$ für Wasserstoff oder Alkyl($C_1$-$C_4$) steht,

$R^4$ für Wasserstoff oder Alkyl($C_1$-$C_4$) steht,

$R^5$ für Wasserstoff, Alkyl($C_1$-$C_4$), Phenyl oder Alkyl($C_1$-$C_3$)thio steht,

$R^6$ für gegebenenfalls durch Fluor, Chlor, Brom, Halogenalkyl($C_1$-$C_3$), Halogenalkoxy($C_1$-$C_3$)-substituiertes Alkyl($C_1$-$C_4$), für gegebenenfalls durch Fluor, Chlor, Brom, Halogenalkyl($C_1$-$C_3$), Halogenalkoxy($C_1$-$C_3$) substituiertes Cycloalkyl($C_3$-$C_6$) oder für den Rest

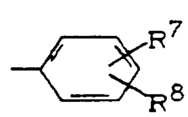

steht,

wobei $R^7$ und $R^8$ gleich oder verschieden sein können und für

Wasserstoff, Fluor, Chlor, Brom, Jod, Alkyl($C_1$-$C_4$), Nitro, Cyano, Halogenalkyl($C_1$-$C_3$), Alkoxy($C_1$-$C_4$), Halogenalkoxy ($C_1$-$C_3$), Alkyl($C_1$-$C_3$)thio, Halogenalkyl($C_1$-$C_3$)thio, gegebenenfalls durch Fluor, Chlor, Brom, Halogenalkyl($C_1$-$C_3$), Alkoxy($C_1$-$C_3$), Alkyl($C_1$-$C_3$) substituiertes Phenoxy, gegebenenfalls durch Fluor, Chlor, Brom, Alkoxy($C_1$-$C_3$), Halogenalkyl($C_1$-$C_3$) substituierts Mono- oder Dialkylamino mit jeweils 1 bis 4 Kohlenstoffatomen im Alkylrest, gegebenenfalls durch Alkyl($C_1$-$C_3$), Alkoxy($C_1$-$C_3$), Fluor, Chlor, Brom, Alkyl($C_1$-$C_3$)thio substituiertes Cycloalkyl($C_3$-$C_6$) stehen oder wobei

$R^7$ und $R^8$ zusammen für eine der folgenden bivalenten Rest stehen:

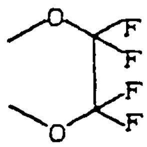 , 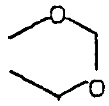 , 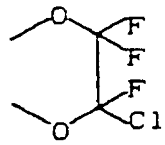 , 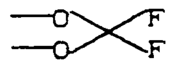 ,

X für Sauerstoff oder Schwefel steht und

Y und Z gleich oder verschieden sein können und für Wasserstoff, Alkyl($C_1$-$C_4$), Fluor, Chlor, Brom, Jod, Halogenalkyl($C_1$-$C_4$), Alkoxy($C_1$-$C_4$), Alkyl($C_1$-$C_4$) thio, Halogenalkoxy($C_1$-$C_3$), Halogenalkyl($C_1$-$C_3$)thio, Alkoxy($C_1$-$C_3$)carbonyl, gegebenenfalls durch Fluor, Chlor, Brom, Alkyl-($C_1$-$C_3$), Alkoxy ($C_1$-$C_3$), Halogenalkyl($C_1$-$C_3$) substituiertes Phenoxy oder Phenylthio, Halogenalkoxy($C_1$-$C_3$)carbonyl, Alkenyl($C_2$-$C_4$)oxy, Alkinyl($C_2$-$C_4$), Alkyl($C_1$-$C_3$) thionyl, Alkyl($C_1$-$C_3$)sulfonyl, Halogenalkyl($C_1$-$C_3$) thionyl, Halogenalkyl($C_1$-$C_3$)sulfonyl, gegebenenfalls durch Halogen, Alkoxy ($C_1$-$C_3$), Halogenalkyl ($C_1$-$C_3$), substituiertes Mono- oder Dialkyl ($C_1$-$C_3$)amino, Nitro, Cyano stehen oder wobei

Y und Z zusammen für gegebenenfalls durch Fluor und/oder Chlor substituiertes 3,4-Methylendioxy oder 3,4-Ethylendioxy stehen.

Die Substituentenbedeutung Halogenalkyl in den Resten Halogenalkyl, Halogenalkoxy, Halogenalkylthio, Halogenalkylthionyl und Halogenalkylsulfonyl enthält vorzugsweise 1 bis 4, insbesondere 1 oder 2 Kohlenstoffatome und vorzugsweise 1 bis 5, insbesondere 1 bis 3 gleiche oder verschiedene Halogenatome, wobei als Halogenatome vorzugsweise Fluor, Chlor und Brom, insbesondere Fluor und Chlor stehen. Beispielhaft seien Trifluormethyl, Chlordifluormethyl, Brommethyl, 2,2,2-Trifluorethyl und Pentafluorethyl genannt.

Im einzelnen seien außer den bei den Herstellungsbeispielen genannten Verbindungen die folgenden substituierten Pyrazolinderivate der allgemeinen Formel (I) genannt:

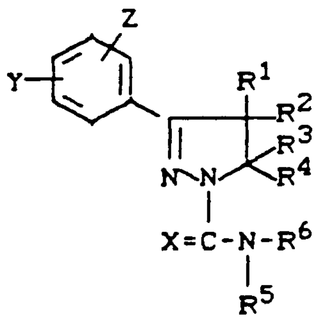

(I)

| Y | Z | X | $R^1$ | $R^2$ | $R^3$ | $R^4$ | $R^5$ | $R^6$ |
|---|---|---|---|---|---|---|---|---|
| H | H | O | 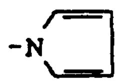 | H | H | H | H | 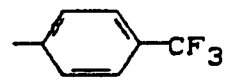 |
| H | H | O | 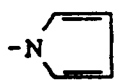 | H | H | H | H | 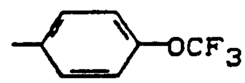 |
| 4-$CF_3$ | H | O | 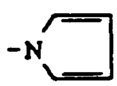 | H | H | H | H | 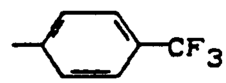 |
| 4-$CF_3$ | H | O | 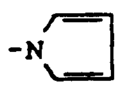 | H | H | H | H | 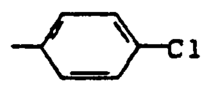 |
| 4-$CF_3$ | H | O | 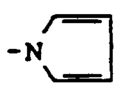 | H | H | H | H | 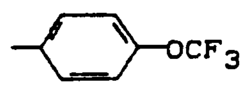 |

| Y | Z | X | $R^1$ | $R^2$ | $R^3$ | $R^4$ | $R^5$ | $R^6$ |
|---|---|---|---|---|---|---|---|---|
| 4-Br | H | O | 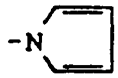 | H | H | H | H | 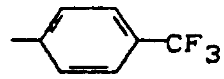 |
| 3-$CF_3$ | H | O | 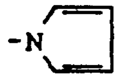 | H | H | H | H | 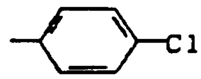 |
| 3-$CF_3$ | H | O | 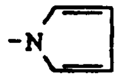 | H | H | H | H | 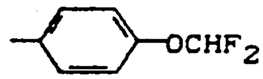 |
| 4-F | H | O | 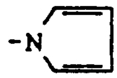 | H | H | H | H | 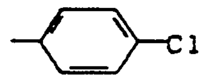 |
| 4-F | H | O | 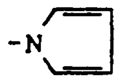 | H | H | H | H | 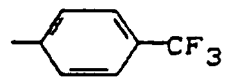 |
| 4-F | H | O | 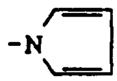 | H | H | H | H | 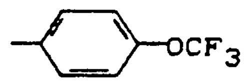 |
| 4-F | H | O | 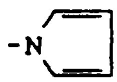 | H | H | H | H | 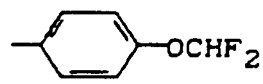 |
| 4-F | H | O | 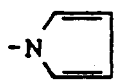 | H | H | H | H | 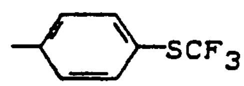 |
| 4-F | H | O | 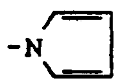 | H | H | H | H | 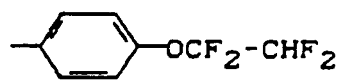 |
| 4-F | H | O | 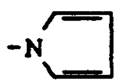 | H | H | H | H | 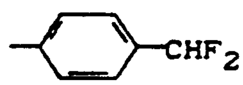 |
| 4-F | H | O | 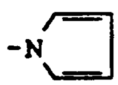 | H | H | H | H | 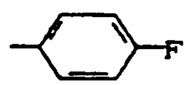 |
| 4-Cl | H | O | 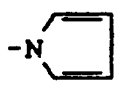 | H | H | H | H | 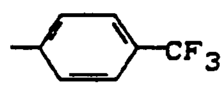 |

| Y | Z | X | $R^1$ | $R^2$ | $R^3$ | $R^4$ | $R^5$ | $R^6$ |
|---|---|---|---|---|---|---|---|---|
| 4-$OCHF_2$ | H | O | 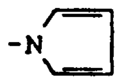 | H | H | H | H | 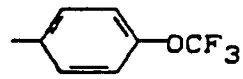 |
| 4-F | H | O | 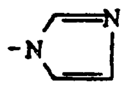 | H | H | H | H | 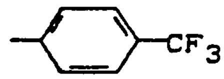 |
| 4-F | H | O | 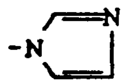 | H | H | H | H | 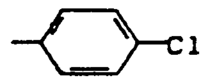 |
| 4-F | H | O | 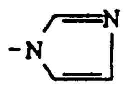 | H | H | H | H | 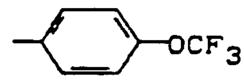 |
| 4-F | H | O | 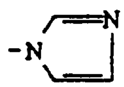 | H | H | H | H | 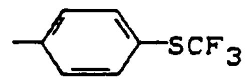 |
| 4-F | H | O | 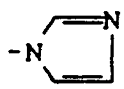 | H | H | H | H | 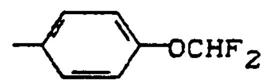 |
| 4-Cl | H | O | 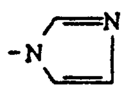 | H | H | H | H | 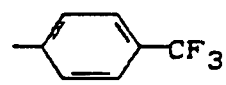 |
| 4-Cl | H | O | 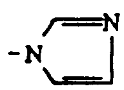 | H | H | H | H | 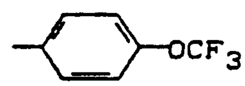 |
| 4-Cl | H | O | 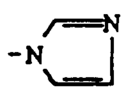 | H | H | H | H | 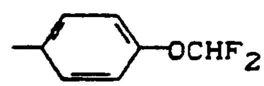 |
| 4-Cl | H | O | 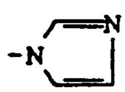 | H | H | H | H | 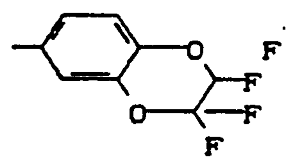 |
| 4-$OCHF_2$ | H | O | 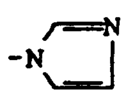 | H | H | H | H | 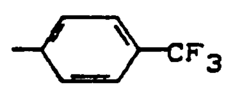 |

| Y | Z | X | $R^1$ | $R^2$ | $R^3$ | $R^4$ | $R^5$ | $R^6$ |
|---|---|---|---|---|---|---|---|---|
| 4-$OCHF_2$ | H | O | 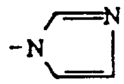 | H | H | H | H | 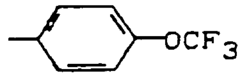 |
| 4-$OCHF_2$ | H | O | 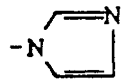 | H | H | H | H | 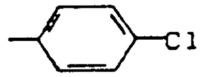 |
| 4-$OCHF_2$ | H | O | 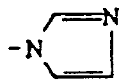 | H | H | H | H | 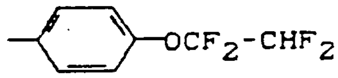 |
| 4-$OCH_2CF_3$ | H | O | 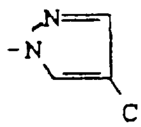 | H | H | H | H | 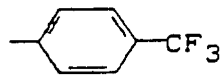 |
| 4-$OCH_2CF_3$ | H | O | 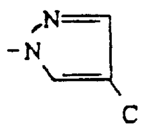 | H | H | H | H | 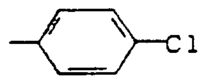 |
| 4-$CF_3$ | H | O | 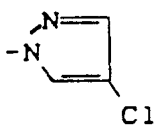 | H | H | H | H | 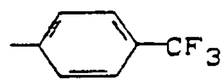 |
| 4-$CF_3$ | H | O | 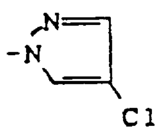 | H | H | H | H | 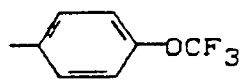 |
| H | H | O | 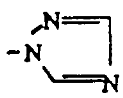 | H | H | H | H | 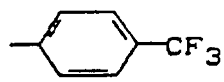 |
| 4-t.-Butyl | H | O | 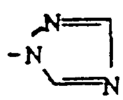 | H | H | H | H | 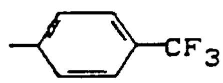 |

| Y | Z | X | $R^1$ | $R^2$ | $R^3$ | $R^4$ | $R^5$ | $R^6$ |
|---|---|---|---|---|---|---|---|---|
| 3-$CF_3$ | H | O | 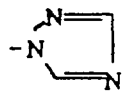 | H | H | H | H | 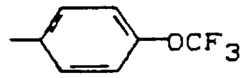 |
| 4-$CH_3$ | H | O | 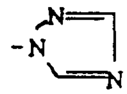 | H | H | H | H | 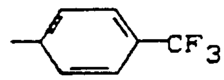 |
| 4-$CH_3$ | H | O | 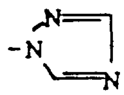 | H | H | H | H | 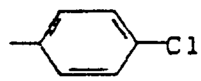 |
| 4-Cl | H | O | 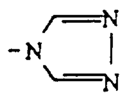 | H | H | H | H | 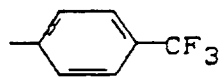 |
| 4-Cl | H | O | 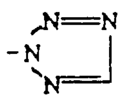 | H | H | H | H | 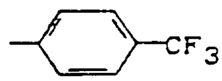 |
| 4-F | H | O | 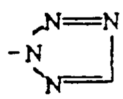 | H | H | H | H | 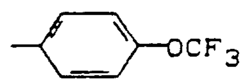 |
| 4-Cl | H | O | 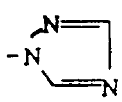 | H | $CH_3$ | H | H | 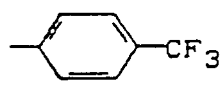 |
| 4-$OCHF_2$ | H | O | 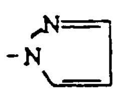 | H | $CH_3$ | H | H | 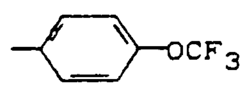 |
| 4-F | H | O | 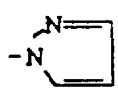 | H | $CH_3$ | $CH_3$ | H | 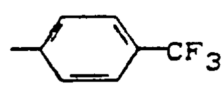 |

Verwendet man beispielsweise

3-(4'-Fluorphenyl)-4-(1"H-4"-chlorpyrazol-1"-yl)-4,5-dihydropyrazol und 4-Difluormethoxyphenylisocyanat als Ausgangsstoffe, so läßt sich der Reaktionsablauf des erfindungsgemäßen Verfahrens a) durch das folgende Formelschema darstellen:

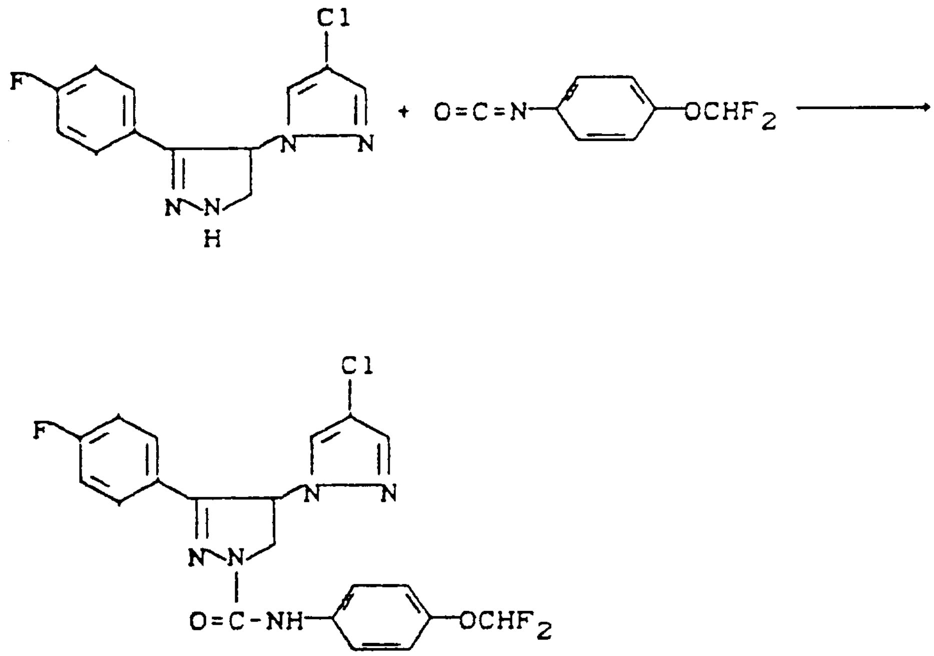

Verwendet man beispielsweise

3-(4-Fluorphenyl)-4-(1H-pyrazol-1-yl)-4,5-dihydro-1-pyrazolcarbonsäure-(4-trifluormethyl-anilid) und 2-Jodpropan als Ausgangsstofe, so kann der Reaktionsablauf des erfindungsgemäßen Verfahrens b) wie folgt dargestellt werden:

1.) Butyl-Lithium

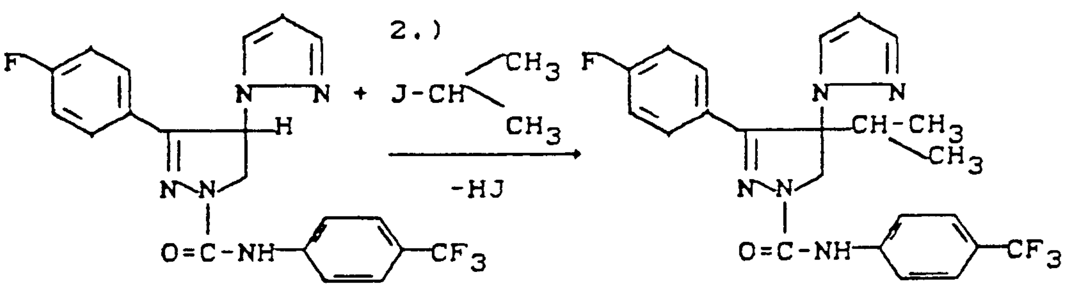


Die zur Durchführung des erfindungsgemäßen Verfahrens (a) als Ausgangsstoffe benötigten Pyrazolinderivate sind durch die Formel (II) allgemein definiert. In dieser Formel (II) stehen $R^1$, $R^2$, $R^3$, $R^4$, Y und Z vorzugsweise für diejenigen Reste, die bereits im Zusammenhang mit der Beschreibung der erfindungsgemäßen Stoffe der Formel (I) als bevorzugt für diese Substituenten genannt wurden. Die Pyrazolinderivate der Formel (II) sind neu.

Sie werden durch Umsetzung von Verbindungen der Formel (VI)

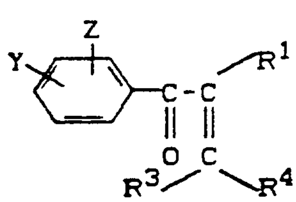

(VI)

in einem polaren organischen Lösungsmittel, vorzugsweise einem Alkanol bei Temperaturen von 20 bis 80 °C insbesondere bei 30 bis 60 °C mit Hydrazin-Hydrat hergestellt:

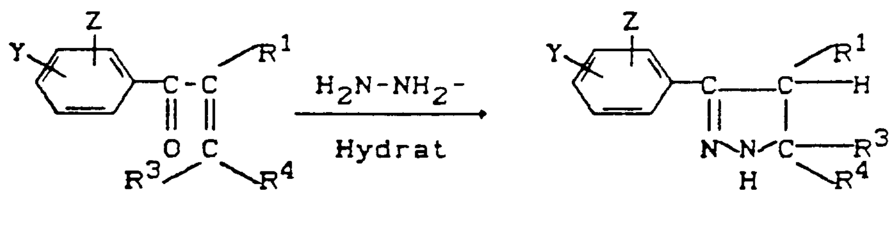


(VI) (II)

In Abhängigkeit von der Bedeutung der Substituenten $R^3$ und $R^4$ ergeben sich dabei folgende Herstellungsvarianten der Ausgangsverbindungen der Formel (VI)

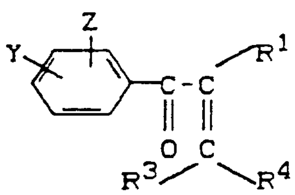

(VI)

a) $R^3$ und $R^4$ in der Formel (VI) stehen für Wasserstoff

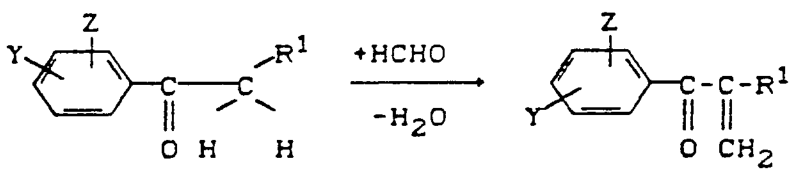


(VIa) (VIb)

Hierbei wird in einem polaren organischen Lösungsmittel, vorzugsweise einem Alkanol und insbesondere in Ethanol oder Methanol mit einer Formalinlösung unter Zusatz geringer Mengen einer organischen Base, insbesondere von Piperidin sowie Zusatz von Eisessig umgesetzt.

b) In der Formel (VI) steht $R^3$ für Alkyl oder Aryl und $R^4$ steht für Wasserstoff:

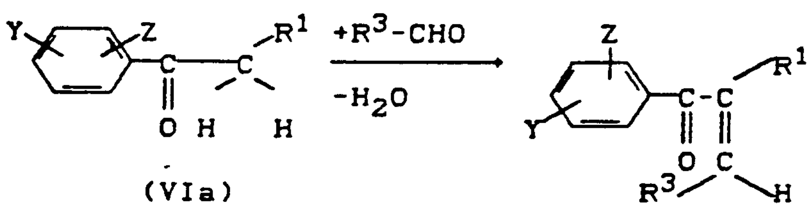


(VIa) (VIc)

Die Verfahrensbedingungen entsprechen denjenigen der Umsetzung mit Formaldehyd.

c) In der Formel stehen $R^3$ und $R^4$ für Alkyl:

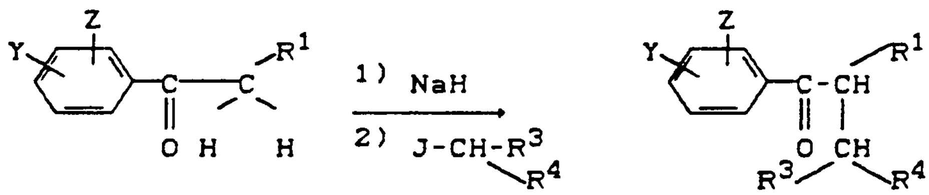


(VIa) (VII)

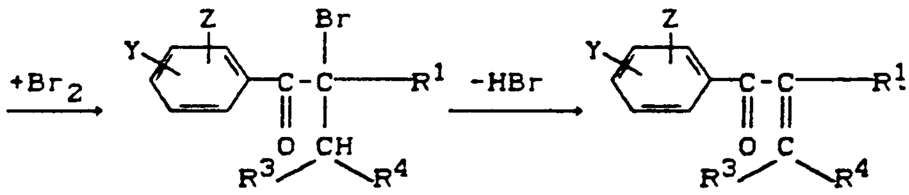


(VIII) (IX)

Hierbei wird die Verbindung (VI) zunächst mit einer starken Base in das Salz übergeführt und anschließend mit einem Halogenid, insbesondere einem Jodid der Formel

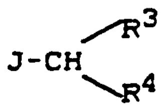

umgesetzt.

Die dabei entstehenden Verbindung der Formel (VII) wird bromiert und anschließend wird durch Zusatz einer Base unter HBr-Eliminierung das Zwischenprodukt der Formel (IX) hergestellt.

Alternativ können Verbindungen der Formel II in der $R^3$ und $R^4$ für Wasserstoff stehen auch hergestellt werden durch Umsetzung von Verbindungen der Formel (XII) in einem polaren Lösungsmittel, vorzugsweise in einem Alkanol bei Temperaturen von 0° bis 60°C mit Hydrazinhydrat:

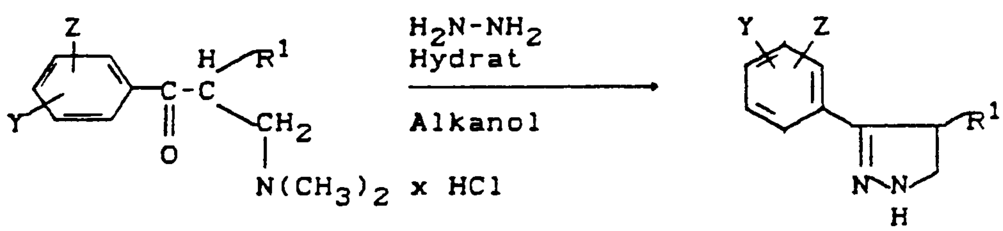


(XII) (II)

Verbindungen der Formel XII können hergestellt werden durch Umsetzung von Verbindungen der Formel VIa in einem Alkanol bei 30 bis 80°C mit Dimethylaminhydrochlorid und Paraformaldehyd und anschließendes Fällen des Salzes mit einem unpolaren Lösungsmittel z.B. Ether:

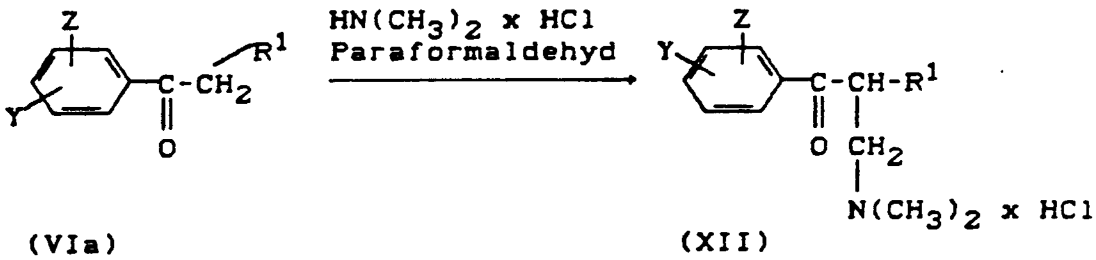


(VIa) (XII)

Die Verbindungen der Formel (VIa) sind teilweise neu. Sie werden durch Umsetzung der Verbindungen

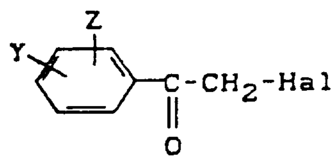

(X)

worin Y und Z die oben angegebene Bedeutung besitzen und Hal für Halogen steht,
mit der Verbindungen $R^1$-H unter Zuhilfenahme einer organischen oder anorganischen Base unter Halogenwasserstoffeliminierung hergestellt. Bei den Verbindungen der Formel (X) handelt es sich um bekannte Stoffe.

Bei der Verfahrensvariante b)
werden Verbindungen der Formel (IV)

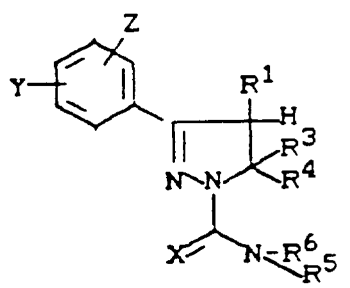

(IV)

in welcher X, Y, Z, $R^1$, $R^3$, $R^4$, $R^5$ und $R^6$ die oben angegebene Bedeutung besitzen mit einer starken Base, vorzugsweise einer metallorganischen Verbindung, insbesondere mit Butyl-Lithium im inerten organischen Lösungsmittel bei Temperaturen von -50 bis 0 °C, insbesondere von -30 °C bis -15 °C, gegebenenfalls in Anwesenheit einer Schutzgasatmosphäre insbesondere Edelgasatmosphäre wie z.B. Argon umgesetzt und anschließend mit einem Halogenid Hal-$R^2$
worin

$R^2$ für Alkyl, gegebenenfalls substituiertes Cycloalkyl, Halogenalkyl, Alkoxycarbonyl oder Halogenalkylthio steht,

bei 0 bis 60 °C, insbesondere bei -10 bis -40 °C umgesetzt und durch Zusatz von Wasser und Extraktion mit Ether in üblicher Weise aufgearbeitet.

Man erhält dann Verbindungen der Formel (XI)

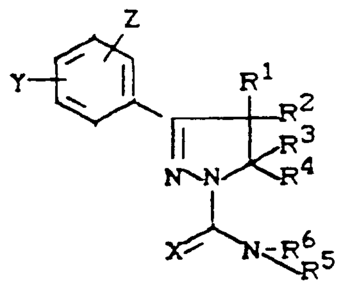

(XI)

wobei X, Y, Z, $R^1$, $R^2$, $R^3$, $R^4$, $R^5$, $R^6$ und $R^9$ die oben angegebenen Bedeutungen besitzen.

Die Wirkstoffe eignen sich zur Bekämpfung von tierischen Schädlingen, vorzugsweise Arthropoden und Nematoden, insbesondere Insekten und Spinnentieren, die in der Landwirtschaft, in der Tierhaltung, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.
Aus der Ordnung der Hymenoptera z.B. Diprion spp., Hoplocampa spp., Lasius spp., Monomorium

pharaonis, Vespa spp.
Aus der Ordnung der Diptera z.B. Aedes spp., Anopheles spp., Culex spp., Drosophila melanogaster, Musca spp., Hydrotaea spp., Haematobia spp., Glossina spp., Melophagus spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp., Ctenocephalides spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Ornithonyssus spp., Eriophyes ribis, Phyllocoptruta oleivora, Boophilus spp., Rhipicephalus spp., Amblyomma spp., Hyalomma spp., Ixodes spp., Dermacentor spp., Haemaphysalis spp., Otobius spp., Psoroptes spp., Chorioptes spp., Sarcoptes spp., Psorergates spp., Demodex spp., Notoedres spp., Tarsonemus spp., Bryobia praetiosa, Panonychus spp., Tetranychus spp..

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer.

Sie sind gegen normalsensible und resistente Arten und Stämme sowie gegen alle parasitierenden und nicht parasitierenden Entwicklungsstadien der Ekto- und Endoparasiten wirksam.

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten, wie beispielsweise gegen die grüne Pfirsichblattlaus (Myzus persicae) oder gegen die Larven des Meerettichblattkäfers (Phaedon cochleariae) oder gegen die Larven der Kohlschabe (Plutella xylostella) oder gegen die Larven der grünen Reiszikade (Nephotettix cincticeps) oder gegen die Larven des Baumwollkapselwurms (Heliothis armigera) oder gegen die Larven des Heerwurms (Spodoptera frugiperda); zur Bekämpfung von pflanzenschädigenden Milben, wie beispielsweise gegen die gemeine Spinnmilbe oder die Bohnenspinnmilbe (Tetranychus urticae) einsetzen.

Darüberhinaus lassen sie sich mit besonders gutem Erfolg zur Bekämpfung von parasitisch lebenden Warmblüterschädlingen, wie beispielsweise gegen die Larven der Goldfliege (Lucilia cuprina), gegen Rinderzecken (Boophilus microplus) oder gegen Räudemilben (Psoroptes ovis) sowie gegen Schaben (Blattella germanica u.a.) einsetzen.

Weiterhin zeigen die erfindungsgemäßen Verbindungen eine Wirksamkeit gegenüber parasitischen Protozoen und zwar insbesondere gegen Coccidien und/oder Plasmodium.

Die Wirkstoffe können in die übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.
Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können.

Die Anwendung der erfindungsgemäßen Wirkstoffe geschieht auf diesem Gebiet in bekannter Weise, wie durch äußerliche Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion sowie ferner durch das "feed-through"-Verfahren. Daneben ist auch eine Anwendung als Formkörper (Halsband, Ohrmarke), sowie eine Anwendung in Form der sogenannten Umgebungsbehandlung möglich.

Die biologische Wirksamkeit der erfindungsgemäßen Verbindungen soll anhand der folgenden Beispiele erläutert werden.

Herstellungsbeispiele

Beispiel 1

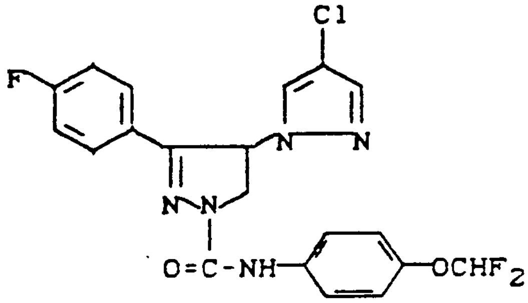

2,64 g (0,01 Mol) 3-(4'-Fluorphenyl)-4-(1"H-4"-chlorpyrazol-1"-yl)-4,5-dihydropyrazol werden in 20 ml wasserfreiem Acetonitril bei Raumtemperatur gelöst und unter Rühren 2 g 4-Difluormethoxyphenylisocyanat zugegeben. Die Mischung wird auf 50 °C erhitzt, dann werden 2 bis 3 Tropfen Triethylamin zugegeben. Anschließend wird bei 50 °C eine Stunde gerührt. Nach dem Abkühlen wird das Lösungsmittel im Vakuum abdestilliert und der Rückstand mit 25 ml Ether versetzt. Die sich nach einiger Zeit abscheidenden Kristalle werden abgesaugt. Man erhält 3,1 g 3(4'-Fluorphenyl)-4-(1"H-4"chlorpyrazol-1"-yl)-4,5-dihydro-1-pyrazolcarbonsäure-(4-difluormethoxy-anilid mit dem Schmelzpunkt 200 °C.

In analoger Weise sind herstellbar:

**Allgemeine Formel**

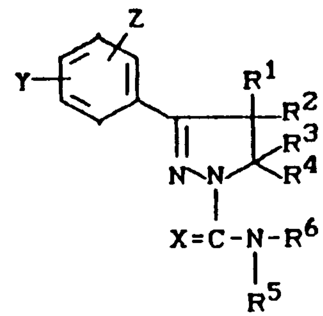

(I)

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 2 | 4-F | H | O | 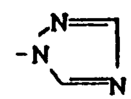 | H | H | H | H | 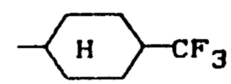 | Öl |
| 3 | 4-Cl | H | O | 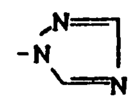 | H | H | H | H | 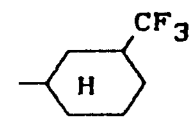 | Öl |
| 4 | 4-Cl | H | O | 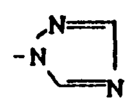 | H | H | H | H | 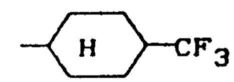 | Öl |
| 5 | 4-Cl | H | O | 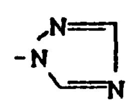 | H | H | H | H | 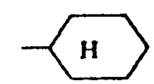 | 149° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 6 | 4-F | H | O | 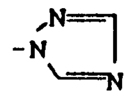 | H | H | H | H | 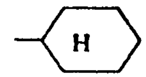 | Öl |
| 7 | 4-Cl | H | O | 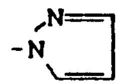 | H | H | H | H | 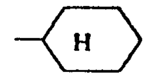 | 195-196° C |
| 8 | 4-Cl | H | O | 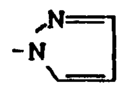 | H | H | H | H | 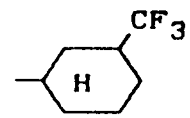 | Öl |
| 9 | 4-Cl | H | O | 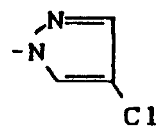 | H | H | H | H | 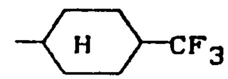 | 222° C |
| 10 | 4-Cl | H | O | 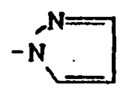 | H | H | H | H | 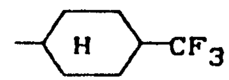 | Öl |
| 11 | 4-Cl | H | O | 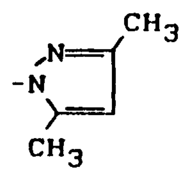 | H | H | H | H | 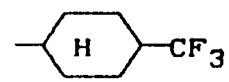 | 176° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 12 | 4-F | H | O | 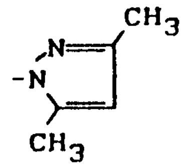 | H | H | H | H | 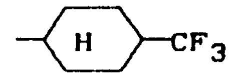 | Öl |
| 13 | 4-F | H | S | 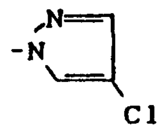 | H | H | H | H | 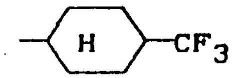 | Öl |
| 14 | 4-Cl | H | S | 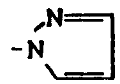 | H | H | H | H | 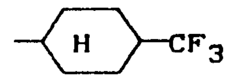 | Öl |
| 15 | 4-Cl | H | S | 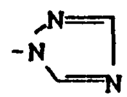 | H | H | H | H | 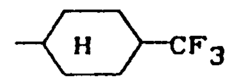 | Öl |
| 16 | 4-$OCHF_2$ | H | O | 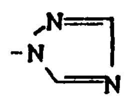 | H | H | H | H | 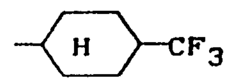 | Öl |
| 17 | 4-$OCHF_2$ | H | O | 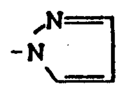 | H | H | H | H | 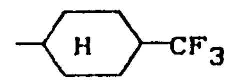 | Öl |
| 17a | 4-F | H | O | 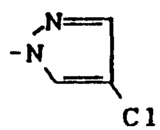 | H | H | H | H | 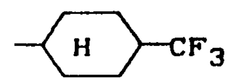 | |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 18 | 4-$OCHF_2$ | H | O | 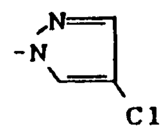 | H | H | H | H | 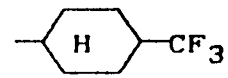 | |
| 19 | 4-$OCHF_2$ | H | S | 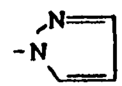 | H | H | H | H | 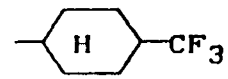 | |
| 20 | 4-$OCHF_2$ | H | O | 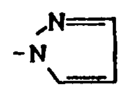 | H | H | H | H | 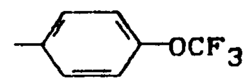 | 170-180° C |
| 21 | 4-Cl | H | O | 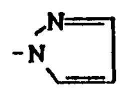 | H | H | H | H | 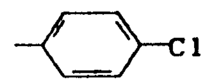 | <250° C |
| 22 | 4-Cl | H | O | 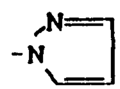 | H | H | H | H | 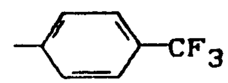 | 225° C |
| 23 | 4-Cl | H | O | 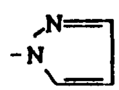 | H | H | H | H | 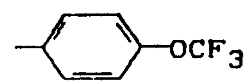 | 229° C |
| 24 | 4-Cl | H | O | 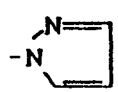 | H | H | H | H | 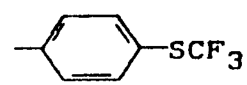 | 233° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 25 | 4-Cl | H | O | 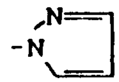 | H | H | H | H | 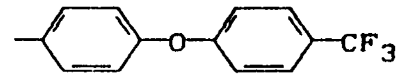 | 236-237° C |
| 26 | 4-Cl | H | O | 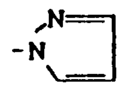 | H | H | H | H | 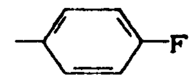 | 185° C |
| 27 | 4-Cl | H | O | 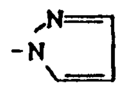 | H | H | H | H | 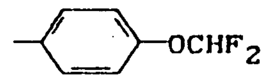 | 224° C |
| 28 | 4-Cl | H | O | 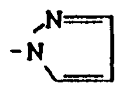 | H | H | H | H | 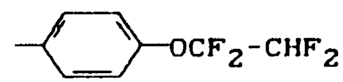 | 225° C |
| 29 | 4-Cl | H | O | 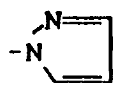 | H | H | H | H | 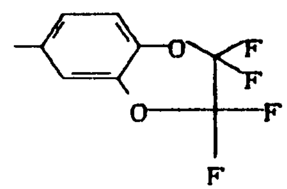 | 227° C |
| 30 | 4-Cl | H | O | 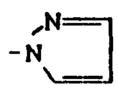 | H | H | H | H | 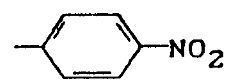 | <250° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 31 | 4-Cl | H | O | 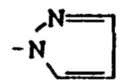 | H | H | H | H | 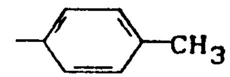 | 235° C |
| 32 | 4-Cl | H | O | 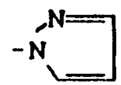 | H | H | H | H | 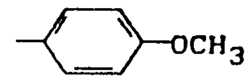 | 245° C |
| 33 | 4-Cl | H | O | 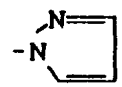 | H | H | H | H | 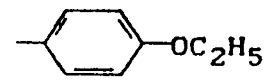 | 215° C |
| 34 | 4-Cl | H | O | 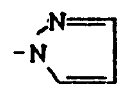 | H | H | H | H | 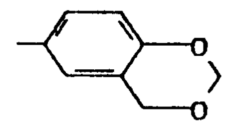 | 170° C |
| 35 | 4-$OCHF_2$ | H | O | 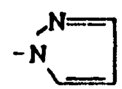 | H | H | H | H | 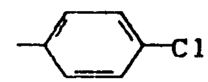 | 170° C |
| 36 | 4-$OCHF_2$ | H | O | 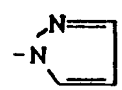 | H | H | H | H | 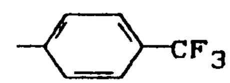 | 175° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 37 | 4-$OCHF_2$ | H | O | 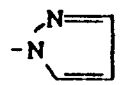 | H | H | H | H | 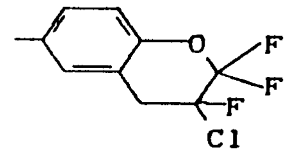 | Harz |
| 38 | 4-$OCHF_2$ | H | O | 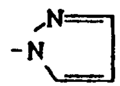 | H | H | H | H | 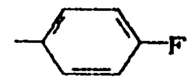 | 195° C |
| 39 | 4-$OCHF_2$ | H | O | 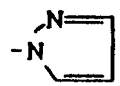 | H | H | H | H | 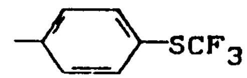 | 219° C |
| 40 | 4-$OCHF_2$ | H | O | 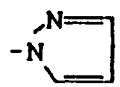 | H | H | H | H | 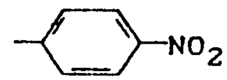 | $<$ 230° C |
| 41 | 4-$OCHF_2$ | H | O | 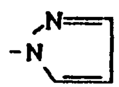 | H | H | H | H | 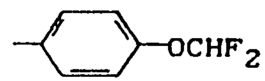 | Harz |
| 42 | 4-F | H | O | 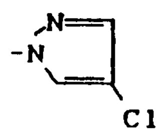 | H | H | H | H | 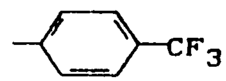 | 211° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 43 | 4-F | H | O | 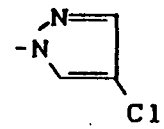 | H | H | H | H | 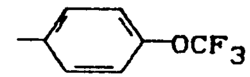 | 227° C |
| 44 | 4-Cl | H | O | 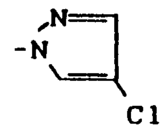 | H | H | H | H | 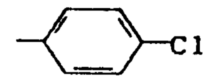 | 216° C |
| 45 | 4-Cl | H | O | 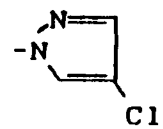 | H | H | H | H | 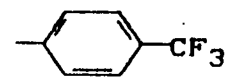 | 213° C |
| 46 | 4-Cl | H | O | 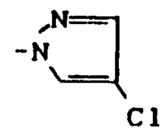 | H | H | H | H | 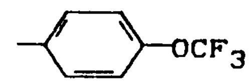 | 217° C |
| 47 | 4-Cl | H | O | 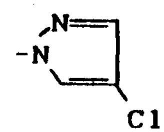 | H | H | H | H | 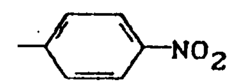 | <250° C |
| 48 | 4-F | H | O | 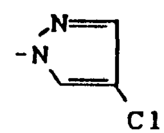 | H | H | H | H | 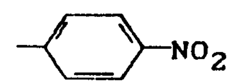 | <250° C |

| Bsp. Nr. | Y | Z. | X | $R^1$ | $R^2$ | $R^3$ | $R^4$ | $R^5$ | $R^6$ | Fp |
|---|---|---|---|---|---|---|---|---|---|---|
| 49 | 4-F | H | O | 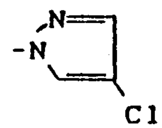 | H | H | H | H | 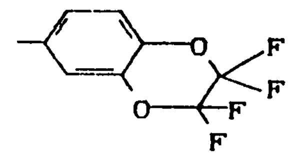 | |
| 50 | 4-F | H | O | 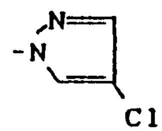 | H | H | H | H | 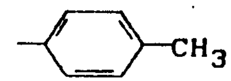 | 235° C |
| 51 | 4-F | H | O | 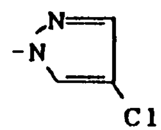 | H | H | H | H | 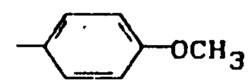 | 195° C |
| 52 | 4-F | H | O | 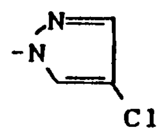 | H | H | H | H | 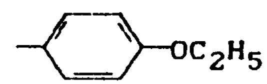 | 196° C |
| 53 | 4-F | H | O | 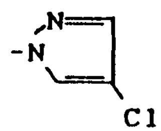 | H | H | H | H | 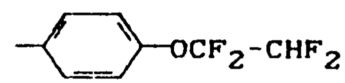 | 209° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 54 | 4-Cl | H | O | 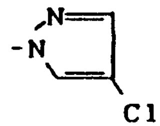 | H | H | H | H | 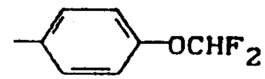 | 179° C |
| 55 | 4-Cl | H | O | 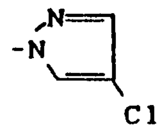 | H | H | H | H | 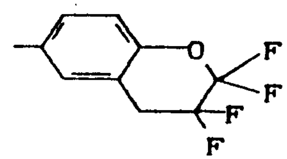 | 186° C |
| 56 | 4-Cl | H | O | 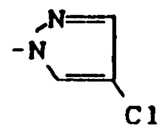 | H | H | H | H | 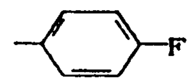 | 194° C |
| 57 | 4-Cl | H | O | 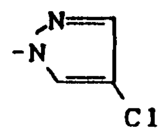 | H | H | H | H | 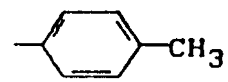 | 245° C |
| 58 | 4-Cl | H | O | 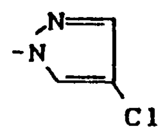 | H | H | H | H | 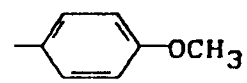 | 222° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 59 | 4-Cl | H | O | 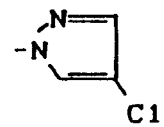 | H | H | H | H | 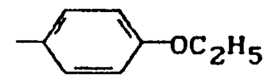 | 241° C |
| 60 | 4-Cl | H | O | 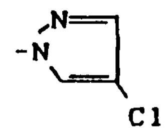 | H | H | H | H | 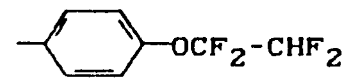 | 239° C |
| 61 | 4-Cl | H | O | 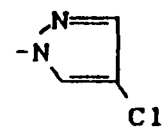 | H | H | H | H | 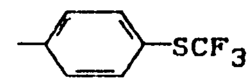 | ‹250° C |
| 62 | 4-$OCHF_2$ | H | O | 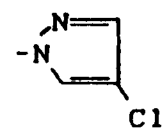 | H | H | H | H | 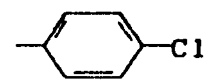 | 207° C |
| 63 | 4-$OCHF_2$ | H | O | 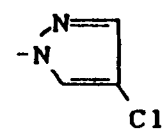 | H | H | H | H | 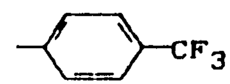 | 192° C |
| 64 | 4-$OCHF_2$ | H | O | 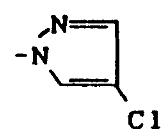 | H | H | H | H | 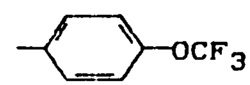 | 170° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 65 | 4-$OCHF_2$ | H | O | 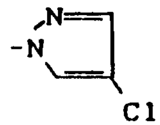 | H | H | H | H | 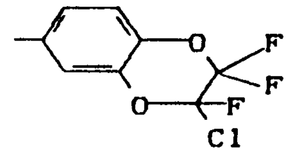 | |
| 66 | 4-Cl | H | O | 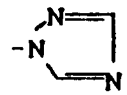 | H | H | H | H | 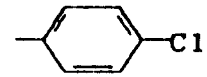 | 230° C |
| 67 | 4-Cl | H | O | 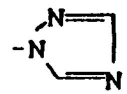 | H | H | H | H | 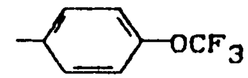 | 198° C |
| 68 | 4-Cl | H | O | 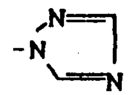 | H | H | H | H | 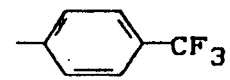 | 228-230° C |
| 69 | 4-$OCHF_2$ | H | O | 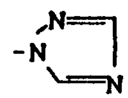 | H | H | H | H | 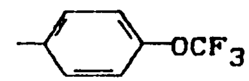 | 215-225° C |
| 70 | 4-Cl | H | O | 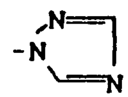 | H | H | H | H | 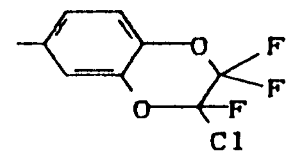 | |

| Bsp. Nr. | Y | Z | X | R¹ | R² | R³ | R⁴ | R⁵ | R⁶ | Fp |
|---|---|---|---|---|---|---|---|---|---|---|
| 71 | 4-Cl | H | O | 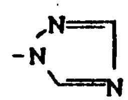 | H | H | H | H | 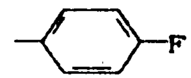 | Öl |
| 72 | 4-F | H | O | 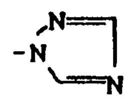 | H | H | H | H | 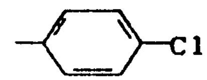 | 191-192° C |
| 73 | 4-F | H | O | 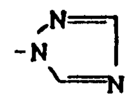 | H | H | H | H | 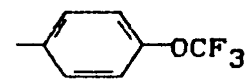 | 215° C |
| 74 | 4-F | H | O | 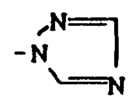 | H | H | H | H | 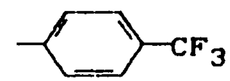 | 217° C |
| 75 | 4-F | H | O | 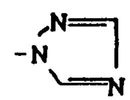 | H | H | H | H | 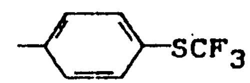 | 246-248° C |
| 76 | 4-F | H | O | 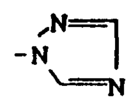 | H | H | H | H | 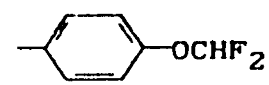 | 212° C |
| 77 | 4-F | H | O | 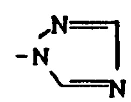 | H | H | H | H | 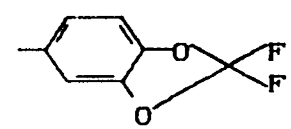 | 226° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 78 | 4-F | H | O | 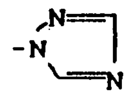 | H | H | H | H | 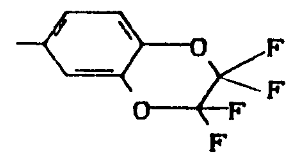 | 225° C |
| 79 | 4-F | H | O | 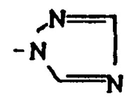 | H | H | H | H | 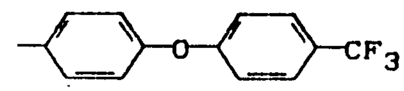 | 200° C |
| 80 | 4-Cl | H | O | 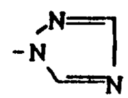 | H | H | H | H | 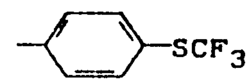 | 235° C |
| 81 | 4-Cl | H | O | 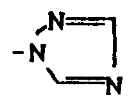 | H | H | H | H | 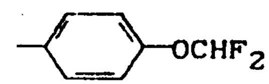 | 231° C |
| 82 | 4-Cl | H | O | 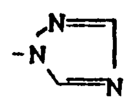 | H | H | H | H | 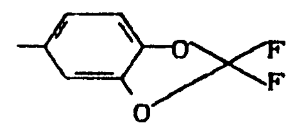 | 233-234° C |
| 83 | 4-Cl | H | O | 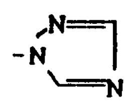 | H | H | H | H | 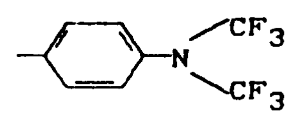 | 227° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 84 | 4-Cl | H | O | 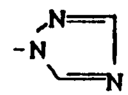 | H | H | H | H | 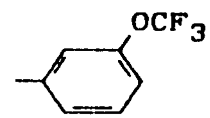 | 239-240° C |
| 85 | 4-Cl | H | O | 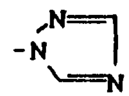 | H | H | H | H | 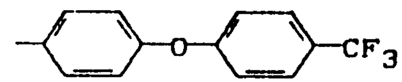 | 214° C |
| 86 | 4-Cl | H | O | 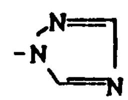 | H | H | H | H | 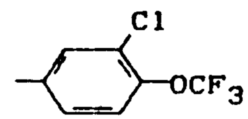 | 212° C |
| 87 | 4-Cl | H | O | 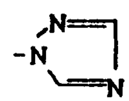 | H | H | H | H | 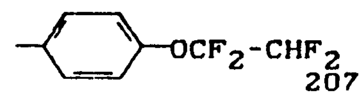 | 207-208° C |
| 88 | 4-Cl | H | O | 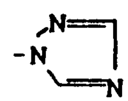 | H | H | H | H | 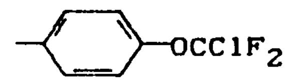 | 212° C |
| 89 | 4-Cl | H | O | 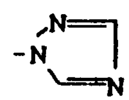 | H | H | H | H | 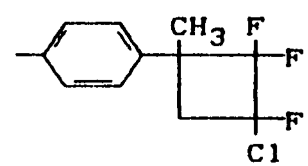 | 109° C |
| 90 | 4-F | H | O | 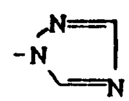 | H | H | H | H | 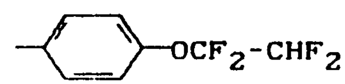 | Öl |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 91 | 4-$OCF_3$ | H | O | 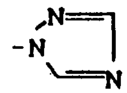 | H | H | H | H | 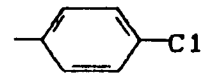 | 215-218° C |
| 92 | 4-Cl | H | O | 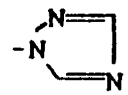 | H | H | H | H | 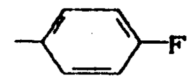 | 135° C |
| 93 | 4-$OCF_3$ | H | O | 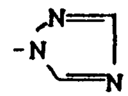 | H | H | H | H | 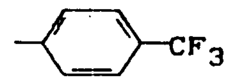 | 250° C |
| 94 | 4-$OCF_3$ | H | O | 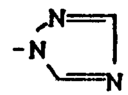 | H | H | H | H | 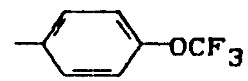 | 230° C |
| 95 | 4-Cl | H | O | 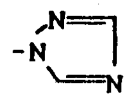 | H | H | H | H | 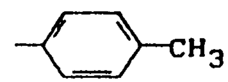 | 250° C |
| 96 | 4-Cl | H | O | 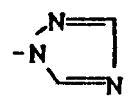 | H | H | H | H | 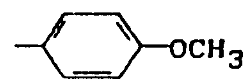 | 186° C |
| 97 | 4-Cl | H | O | 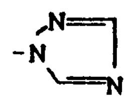 | H | H | H | H | 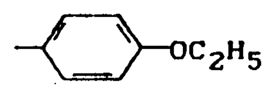 | 210° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 98 | 4-$OCF_3$ | H | O | 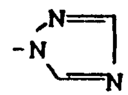 | H | H | H | H | 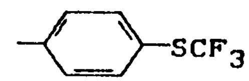 | Öl |
| 99 | 4-Cl | H | O | 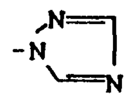 | H | H | H | H | 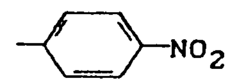 | <250° C |
| 100 | 4-Cl | H | O | 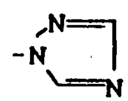 | H | H | H | H | 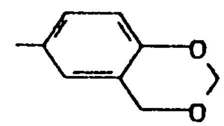 | 210° C |
| 101 | 4-$OCHF_2$ | H | O | 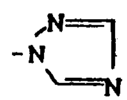 | H | H | H | H | 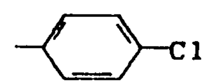 | 216° C |
| 102 | 4-$OCHF_2$ | H | O | 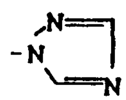 | H | H | H | H | 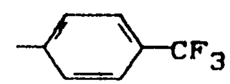 | 246° C |
| 103 | 4-$OCHF_2$ | H | O | 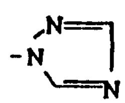 | H | H | H | H | 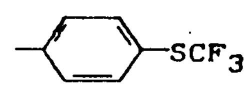 | 231° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 104 | 4-$OCHF_2$ | H | O | 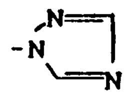 | H | H | H | H | 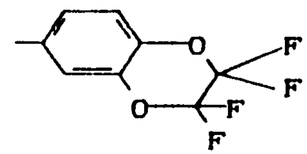 | 214° C |
| 105 | 4-Cl | H | O | 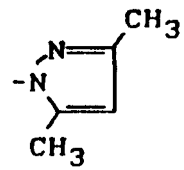 | H | H | H | H | 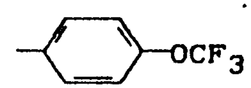 | ‹250° C |
| 106 | 4-Cl | H | O | 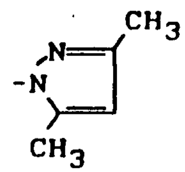 | H | H | H | H | 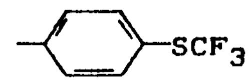 | ‹250° C |
| 107 | 4-F | H | O | 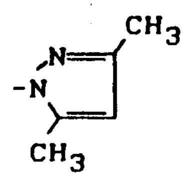 | H | H | H | H | 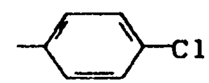 | 234° C |
| 108 | 4-F | H | O | 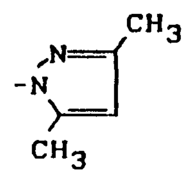 | H | H | H | H | 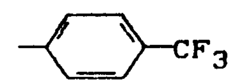 | Öl |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 109 | 4-F | H | O | 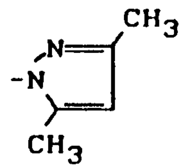 | H | H | H | H | 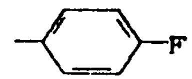 | 240° C |
| 110 | 4-F | H | O | 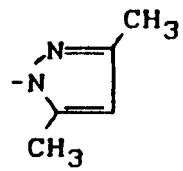 | H | H | H | H | 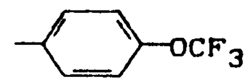 | 250° C |
| 111 | 4-F | H | O | 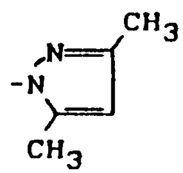 | H | H | H | H | 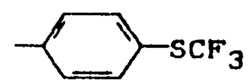 | 260° C |
| 112 | 4-Cl | H | O | 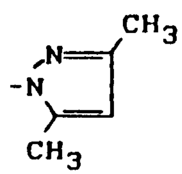 | H | H | H | H | 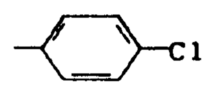 | 245° C |
| 113 | 4-Cl | H | O | 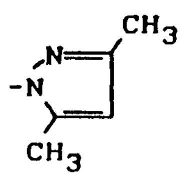 | H | H | H | H | 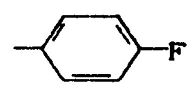 | 247° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 114 | 4-Cl | H | O | 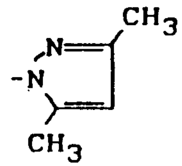 | H | H | H | H | 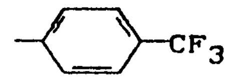 | 250° C |
| 115 | 4-Cl | H | O | 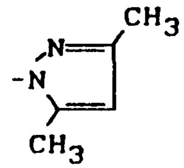 | H | H | H | H | 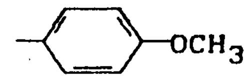 | 250° C |
| 116 | 4-Cl | H | O | 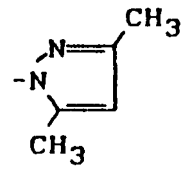 | H | H | H | H | 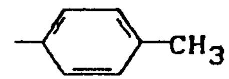 | 243° C |
| 117 | 4-F | H | O | 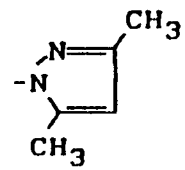 | H | H | H | H | 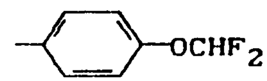 | <250° C |
| 118 | 4-F | H | O | 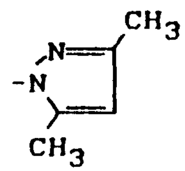 | H | H | H | H | 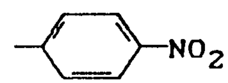 | <250° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 119 | 4-Cl | H | O | 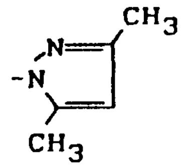 | H | H | H | H | 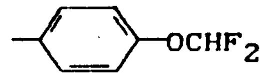 | 224° C |
| 120 | 4-F | H | O | 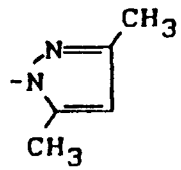 | H | H | H | H | 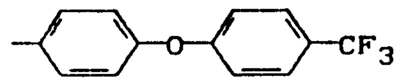 | <250° C |
| 121 | 4-F | H | O | 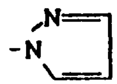 | H | H | H | H | 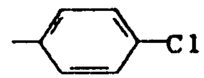 | 201-202° C |
| 122 | 4-F | H | O | 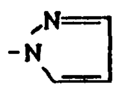 | H | H | H | H | 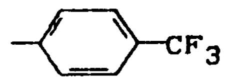 | 201-202° C |
| 123 | 4-F | H | O | 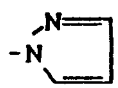 | H | H | H | H | 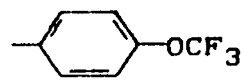 | 207-208° C |
| 124 | 4-F | H | O | 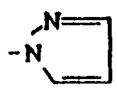 | H | H | H | H | 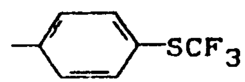 | 220-222° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 125 | 4-F | H | O | 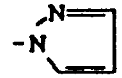 | H | H | H | H | 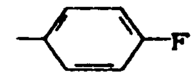 | 193-194° C |
| 127 | 4-F | H | O | 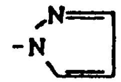 | H | H | H | H | 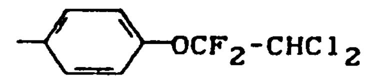 | 201° C |
| 128 | 4-F | H | O | 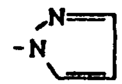 | H | H | H | H | 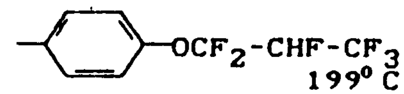 | 199° C |
| 129 | 4-$OCF_2$-CHFCl | H | O | 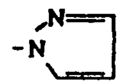 | H | H | H | H | 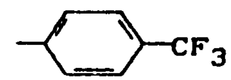 | |
| 130 | 4-$OCF_2$-CHFCl | H | O | 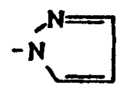 | H | H | H | H | 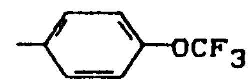 | |
| 131 | 4-$OCF_2$-CHF-$CF_3$ | H | O | 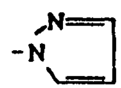 | H | H | H | H | 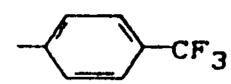 | |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 132 | H | H | O | 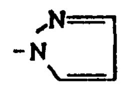 | H | H | H | H | 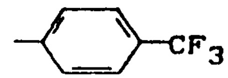 | |
| 133 | 4-Br | H | O | 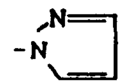 | H | H | H | H | 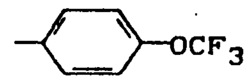 | |
| 134 | 4-F | H | O | 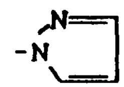 | H | H | H | H | 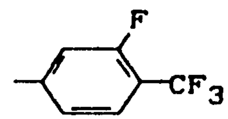 | 218° C |
| 135 | 4-F | H | O | 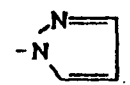 | H | H | H | H | 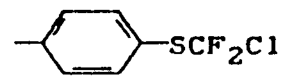 | 234° C |
| 136 | 4-F | H | O | 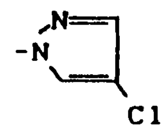 | H | H | H | H | 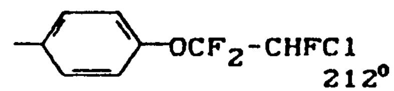 | 212° C |
| 137 | 4-F | H | O | 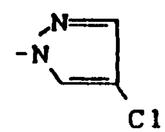 | H | H | H | H | 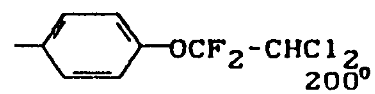 | 200° C |
| 138 | 4-F | H | O | 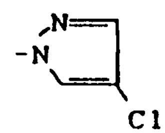 | H | H | H | H | 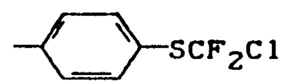 | 212° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 139 | 4-F | H | O | 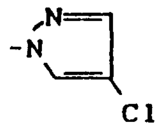 | H | H | H | H | 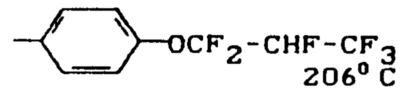 | 206° C |
| 140 | 4-F | H | O | 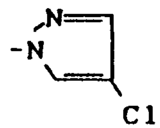 | H | H | H | H | 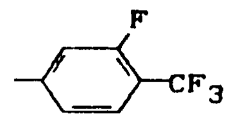 | 202° C |
| 141 | 4-$OCH_2CF_3$ | H | O | 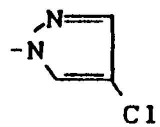 | H | H | H | H | 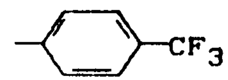 | |
| 142 | 4-$OCF_2$-CHFCl | H | O | 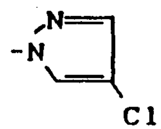 | H | H | H | H | 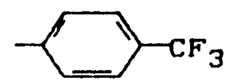 | |
| 143 | 4-$OCF_2$-CHF-$CF_3$ | H | O | 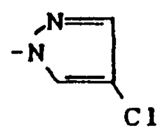 | H | H | H | H | 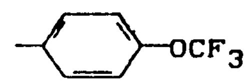 | |
| 144 | 4-$OCF_2Cl$ | H | O | 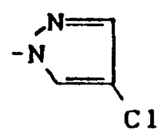 | H | H | H | H | 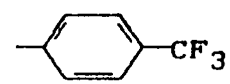 | |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 145 | 4-F | H | O | 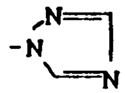 | H | H | H | H | 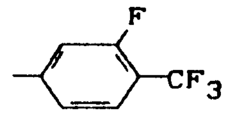 | 217° C |
| 146 | 4-F | H | O | 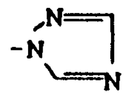 | H | H | H | H | 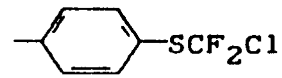 | 202° C |
| 147 | 4-Cl | H | O | 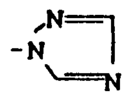 | H | H | H | H | 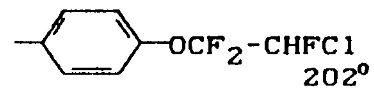 | 202° C |
| 148 | 4-Cl | H | O | 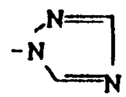 | H | H | H | H | 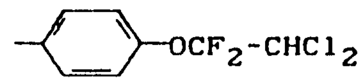 | 199° C |
| 149 | 4-Cl | H | O | 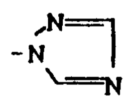 | H | H | H | H | 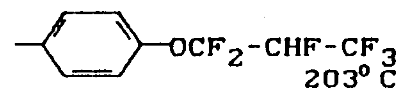 | 203° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 150 | 4-Cl | H | O | 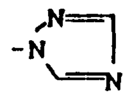 | H | H | H | H | 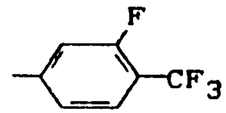 | 224° C |
| 151 | 4-$OCF_2$-CHFCl | H | O | 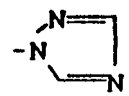 | H | H | H | H | 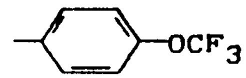 | |
| 152 | 4-Cl | H | O | 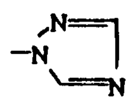 | H | H | H | H | 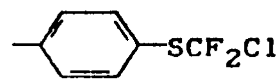 | 214° C |
| 153 | 4-$OCHF_2$ | H | O | 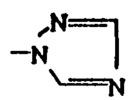 | H | H | H | H | 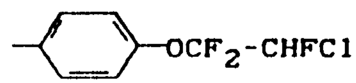 | 206° C |
| 154 | 4-$OCHF_2$ | H | O | 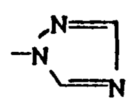 | H | H | H | H | 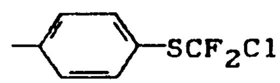 | 226° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 155 | 4-$OCHF_2$ | H | O | 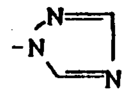 | H | H | H | H | 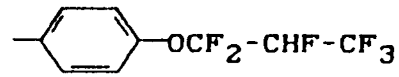 | 205° C |
| 156 | 4-Br | H | O | 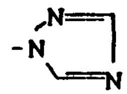 | H | H | H | H | 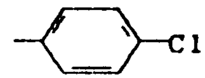 | 224° C |
| 157 | 4-Br | H | O | 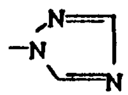 | H | H | H | H | 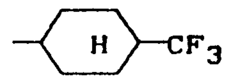 | Harz |
| 158 | 4-Br | H | O | 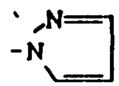 | H | H | H | H | 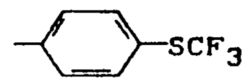 | 211° C |
| 159 | 4-Br | H | O | 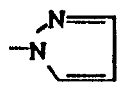 | H | H | H | H | 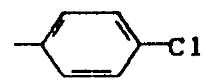 | 187° C |
| 160 | 4-Br | H | O | 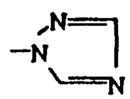 | H | H | H | H | 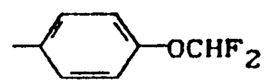 | 225° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 161 | 4-Br | H | O | 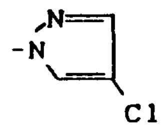 | H | H | H | H | 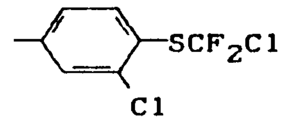 | 215° C |
| 162 | 4-Br | H | O | 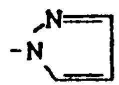 | H | H | H | H | 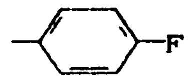 | 216° C |
| 163 | 4-Br | H | O | 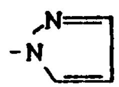 | H | H | H | H | 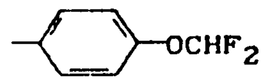 | 216° C |
| 164 | 4-Br | H | O | 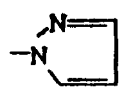 | H | H | H | H | 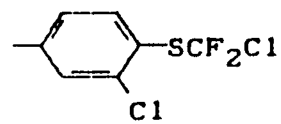 | |
| 165 | 4-Br | H | S | 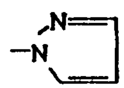 | H | H | H | H | 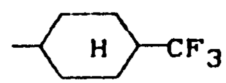 | 161° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 166 | 4-Cl | H | O | 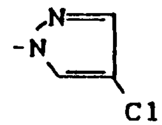 | H | H | H | H | $-CH_2-CH_2-O-CH_2-CF_3$ | |
| 167 | $-OCHF_2$ | H | O | 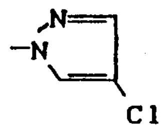 | H | H | H | H | $-CH_2-CH_2-O-CH_2-CF_3$ | |
| 168 | 4-Cl | H | O | 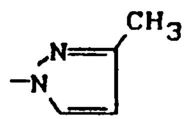 | H | H | H | H | 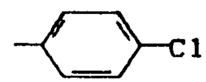 | |
| 169 | $-OCHF_2$ | H | O | 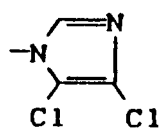 | H | H | H | H | 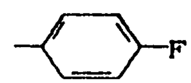 | |
| 170 | 4-$OCF_2$-CHFCl | H | O | 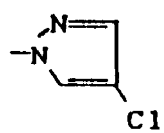 | H | H | H | H | 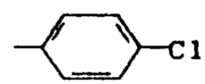 | 200° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 171 | 4-$OCF_2$-CHFCl | H | O | 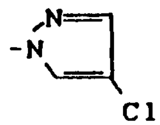 | H | H | H | H | 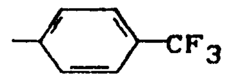 | 210° C |
| 172 | 4-$OCF_2$-CHFCl | H | O | 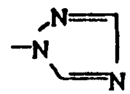 | H | H | H | H | 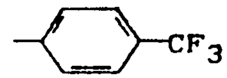 | 248° C |
| 173 | 4-$OCF_2$-CHFCl | H | O | 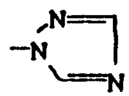 | H | H | H | H | 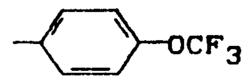 | |
| 174 | 4-Br | H | O | 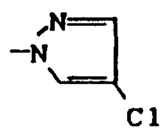 | H | H | H | H | 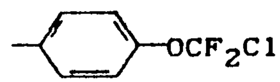 | 223° C |
| 175 | 4-$OCHF_2$ | H | O | 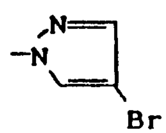 | H | H | H | H | 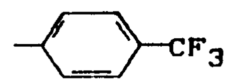 | 152° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 176 | 4-$OCHF_2$ | H | O | 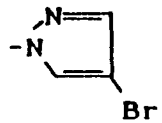 | H | H | H | H | 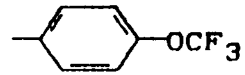 | 211° C |
| 177 | 4-$OCHF_2$ | H | O | 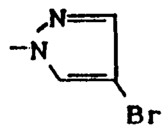 | H | H | H | H | 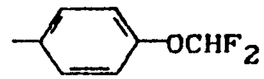 | 169° C |
| 178 | 4-$OCHF_2$ | H | O | 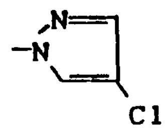 | H | H | H | H | 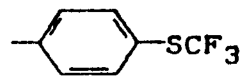 | 226° |
| 179 | 4-Br | H | O | 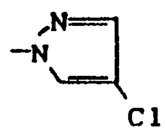 | H | H | H | H | 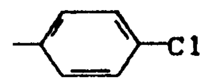 | |
| 180 | 4-Br | H | O | 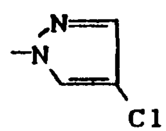 | H | H | H | H | 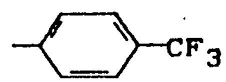 | 202° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 181 | 4-Br | H | O | 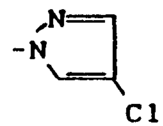 | H | H | H | H | 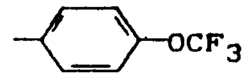 | 249° C |
| 182 | 4-OCHF$_2$ | H | O | 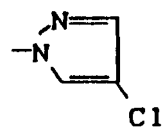 | H | H | H | H | 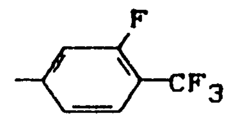 | 201° C |
| 183 | H | H | O | 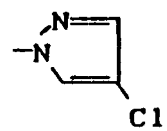 | H | H | H | H | 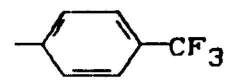 | 227° |
| 184 | 4-Cl | H | O | 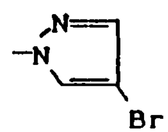 | H | H | H | H | 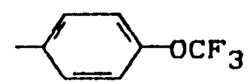 | 243° C |
| 185 | H | H | O | 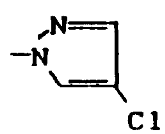 | H | H | H | H | 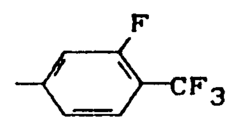 | 219° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 186 | 4-Br | H | O | 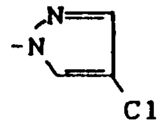 | H | H | H | H | 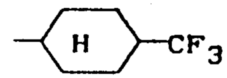 | |
| 187 | 4-Br | H | O | 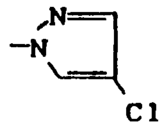 | H | H | H | H | 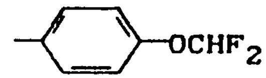 | 208° C |
| 188 | 4-Br | H | O | 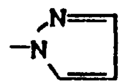 | H | H | H | H | 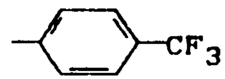 | 224° |
| 189 | 4-Br | H | O | 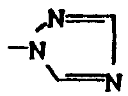 | H | H | H | H | 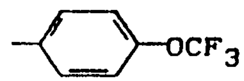 | |
| 190 | 4-Br | H | O | 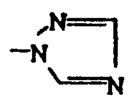 | H | H | H | H | 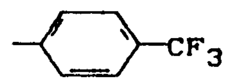 | 249° C |
| 191 | 4-$OCHF_2$ | H | O | 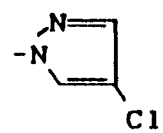 | H | H | H | H | 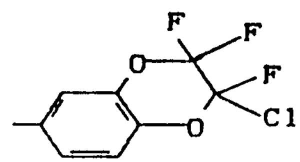 | |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 192 | 4-$OCHF_2$ | H | O | 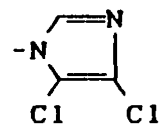 | H | H | H | H | 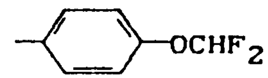 | |
| 193 | 4-$OCHF_2$ | H | O | 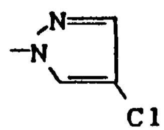 | H | H | H | H | 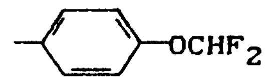 | 180° C |
| 194 | 4-$OCHF_2$ | H | O | 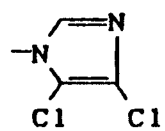 | H | H | H | H | 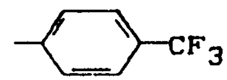 | |
| 195 | 4-$OCHF_2$ | H | O | 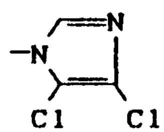 | H | H | H | H | 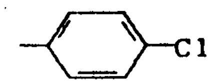 | |
| 196 | 4-$OCHF_2$ | H | O | 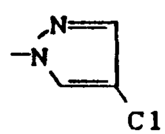 | H | H | H | H | 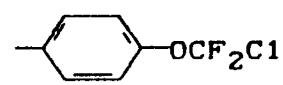 | 201° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 197 | 4-Cl | H | O | 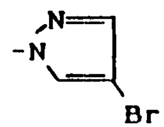 | H | H | H | H | 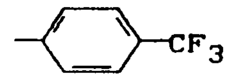 | 242° C |
| 198 | 4-Cl | H | O | 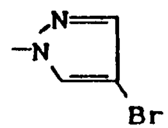 | H | H | H | H | 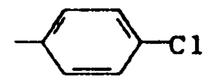 | 212° C |
| 199 | 4-$OCHF_2$ | H | O | 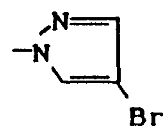 | H | H | H | H | 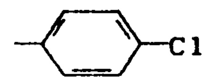 | 219° C |
| 200 | 4-Br | H | O | 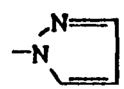 | H | H | H | H | 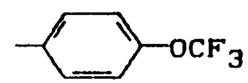 | 219° C |
| 201 | 4-Br | H | O | 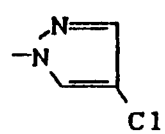 | H | H | H | H | 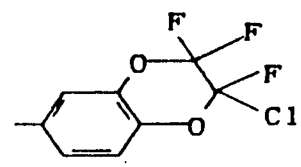 | 217° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 202 | 4-$OCHF_2$ | H | O | 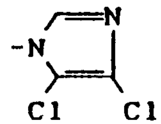 | H | H | H | H | 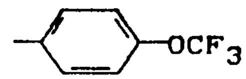 | |
| 203 | 4-Br | H | O | 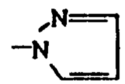 | H | H | H | H | 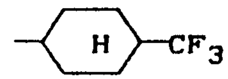 | Öl |
| 204 | H | H | O | 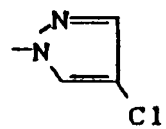 | H | H | H | H | 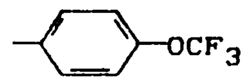 | 218° C |
| 205 | H | H | O | 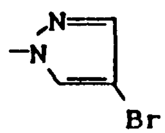 | H | H | H | H | 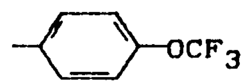 | 223° C |
| 206 | H | H | O | 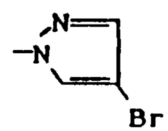 | H | H | H | H | 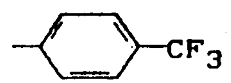 | 245° C |
| 206a | 4-F | H | O | 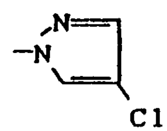 | H | H | H | H | 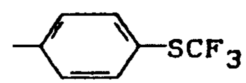 | |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 207 | 4-$OCH_3$ | H | O | 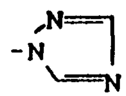 | H | H | H | H | 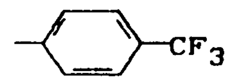 | 221° C |
| 208 | 4-$OCH_3$ | H | O | 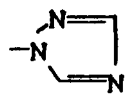 | H | H | H | H | 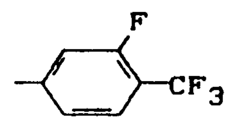 | 238° C |
| 209 | 4-$OCHF_2$ | H | O | 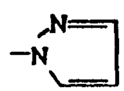 | H | H | H | H | 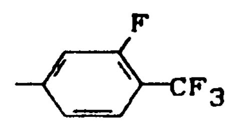 | 252° C |
| 210 | 4-$OCH_3$ | H | O | 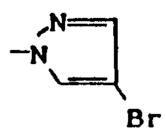 | H | H | H | H | 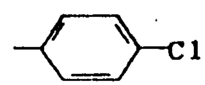 | 221° C |
| 211 | 4-$OCH_3$ | H | O | 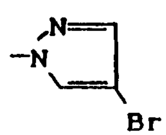 | H | H | H | H | 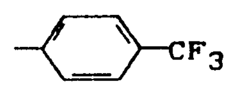 | 250° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 212 | 4-$OCH_3$ | H | O | 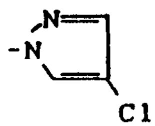 | H | H | H | H | 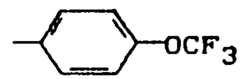 | 210° C |
| 213 | 4-$OCH_3$ | H | O | 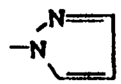 | H | H | H | H | 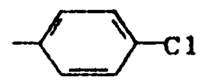 | 201° C |
| 214 | 4-$OCH_3$ | H | O | 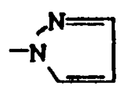 | H | H | H | H | 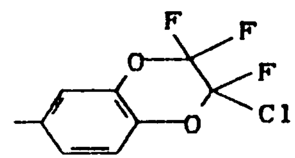 | 186° C |
| 215 | H | H | O | 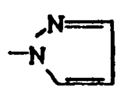 | H | H | H | H | 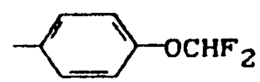 | 212° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 216 | H | H | O | 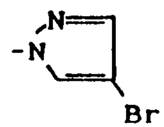 | H | H | H | H | 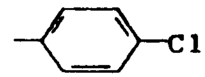 | 233° C |
| 217 | H | H | O | 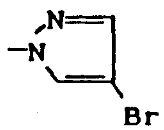 | H | H | H | H | 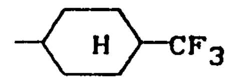 | 236° C |
| 218 | H | H | O | 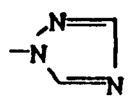 | H | H | H | H | 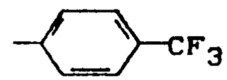 | 215° C |
| 219 | H | H | O | 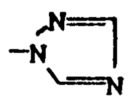 | H | H | H | H | 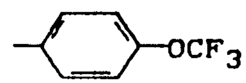 | 245° C |
| 220 | 4-$OC_2H_5$ | H | O | 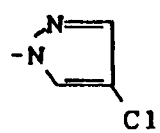 | H | H | H | H | 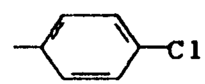 | 179° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 221 | $4-OC_2H_5$ | H | O | 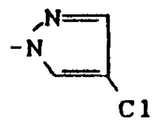 | H | H | H | H | 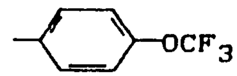 | 153° C |
| 222 | $4-OC_2H_5$ | H | O | 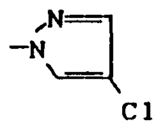 | H | H | H | H | 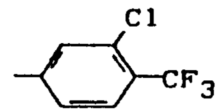 | 176° C |
| 223 | $3-CH_3$ | H | O | 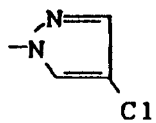 | H | H | H | H | 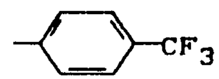 | 224° C |
| 224 | $3-CH_3$ | H | O | 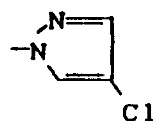 | H | H | H | H | 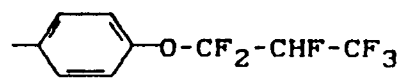 | 190° C |
| 225 | 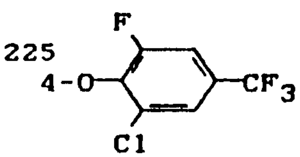 | H | O | 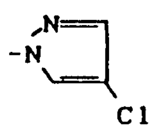 | H | H | H | H | 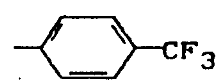 | 208° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 226 | 4-Cl | H | O | 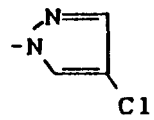 | H | H | H | H | 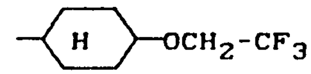 | 164° C |
| 227 | $4-OCHF_2$ | H | O | 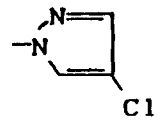 | H | H | H | H | 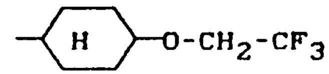 | Öl |
| 228 | 4-Cl | H | O | 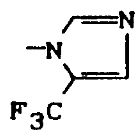 | H | H | H | H | 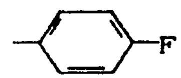 | 197° C |
| 229 | 4-Cl | H | O | 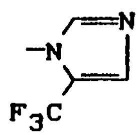 | H | H | H | H | 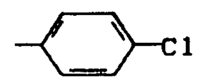 | 215° C |
| 230 | 4-Cl | H | O | 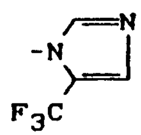 | H | H | H | H | 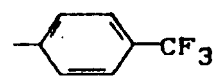 | 248° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 231 | 4-Cl | H | O | 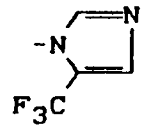 | H | H | H | H | 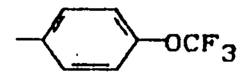 | 188° C |
| 232 | 4-Cl | H | O | 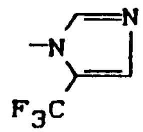 | H | H | H | H | 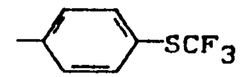 | 227° C |
| 233 | 4-Cl | H | O | 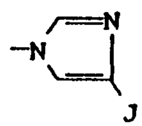 | H | H | H | H | 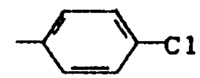 | 234° C |
| 234 | 4-Cl | H | O | 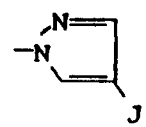 | H | H | H | H | 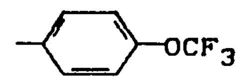 | 232° C |
| 235 | 4-Cl | H | O | 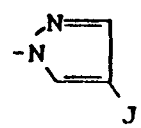 | H | H | H | H | 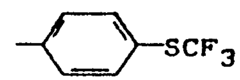 | 218° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 236 | 4-Cl | H | O | 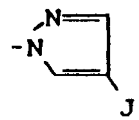 | H | H | H | H | 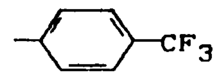 | 188° C |
| 237 | 4-Cl | H | O | 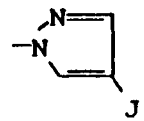 | H | H | H | H | 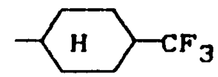 | 184° C |
| 238 | H | H | O | 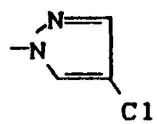 | H | H | H | H | 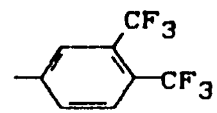 | 234° C |
| 239 | 4-Cl | H | O | 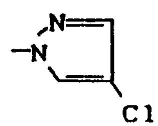 | H | H | H | H | 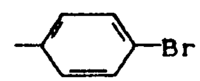 | 217° C |
| 240 | 4-$OCHF_2$ | H | O | 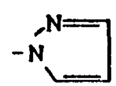 | H | H | H | H | 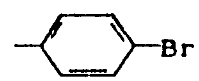 | 224° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 241 | 4-F | H | O | 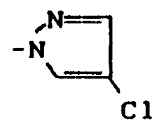 | H | H | H | H | 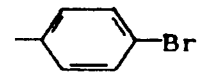 | 195° C |
| 242 | 4-Cl | H | O | 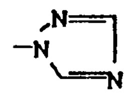 | H | H | H | H | 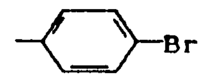 | 205° C |
| 243 | 4-$OCHF_2$ | H | O | 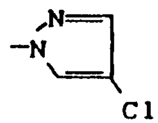 | H | H | H | H | 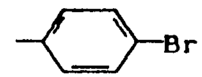 | 188° C |
| 244 | H | H | O | 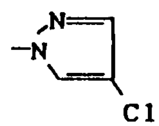 | H | H | H | H | 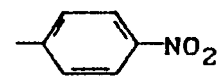 | >250° C |
| 245 | H | H | O | 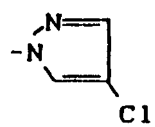 | H | H | H | H | 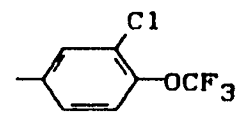 | 180° C |
| 245a | H | H | O | 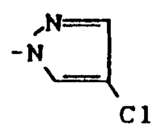 | H | H | H | H | 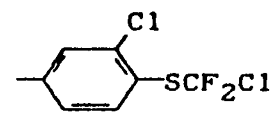 | |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 246 | H | H | O | 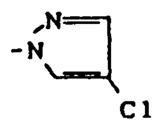 | H | H | H | H | 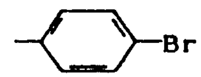 | 201° C |
| 249 | 4-Br | H | O | 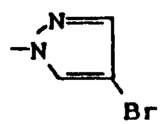 | H | H | H | H | 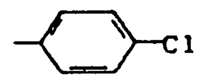 | 215° C |
| 250 | 4-Br | H | O | 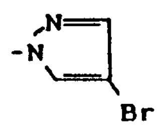 | H | H | H | H | 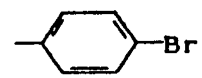 | 225° C |
| 250a | H | H | O | 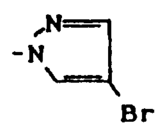 | H | H | H | H | 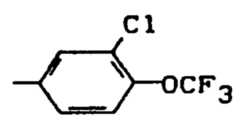 | |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 251 | 4-Br | H | O | 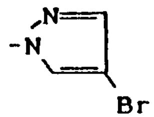 | H | H | H | H | 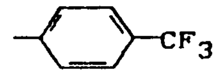 | 222° C |
| 252 | 4-Br | H | O | 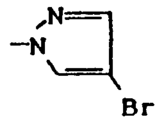 | H | H | H | H | 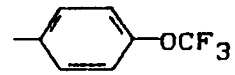 | 235° C |
| 252 | 4-Br | H | O | 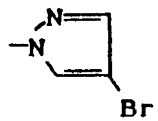 | H | H | H | H | 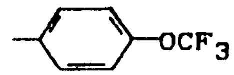 | 235° C |
| 253 | 3,4 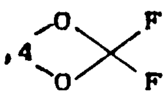 | | O | 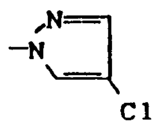 | H | H | H | H | 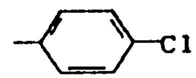 | ›250° C |
| 254 | 3,4 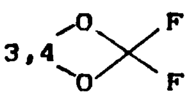 | | O | 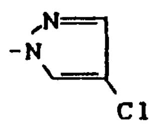 | H | H | H | H | 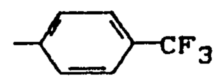 | 199° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 255 | 3,4 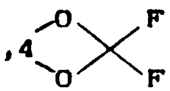 | | O | 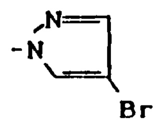 | H | H | H | H | 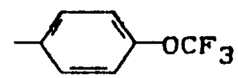 | 178° C |
| 256 | 4-$OCHF_2$ | H | O | 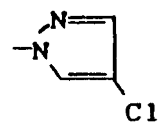 | H | H | H | H | 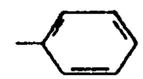 | |
| 257 | H | H | O | 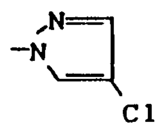 | H | H | H | H | 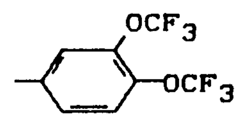 | 170° C |
| 258 | H | H | O | 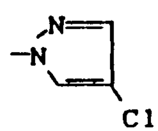 | H | H | H | H | 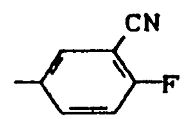 | 235° C |
| 259 | H | H | O | 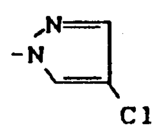 | H | H | H | H | 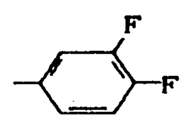 | 208° C |
| 259a | 3,4 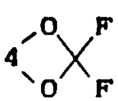 | H | O | 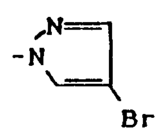 | H | H | H | H | 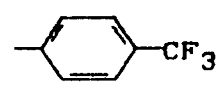 | 208° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 260 | 4-F | H | O | 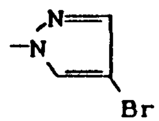 | H | H | H | H | 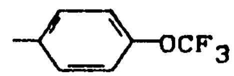 | 222° C |
| 261 | H | H | O | 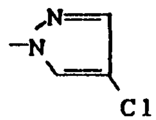 | H | H | H | H | 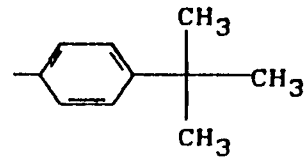 | 198° C |
| 262 | 4-$OCHF_2$ | H | O | 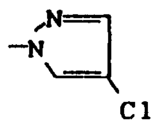 | H | H | H | H | 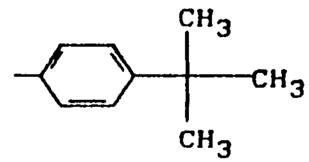 | 235° C |
| 263 | 4-Cl | H | O | 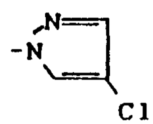 | H | H | H | H | 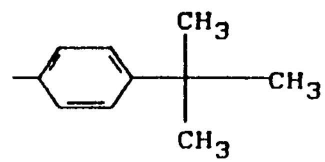 | 192° C |
| 264 | 3,4-$OCH_3$ | H | O | 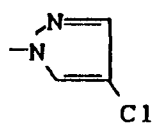 | H | H | H | H | 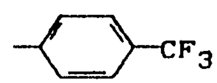 | 216° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 265 | 3-Cl | H | O | 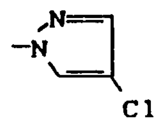 | H | H | H | H | 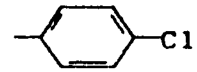 | 192° C |
| 266 | 3-Cl | H | O | 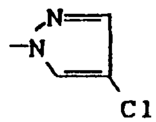 | H | H | H | H | 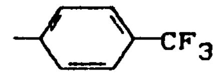 | 195° C |
| 267 | 4-$OCF_3$ | H | O | 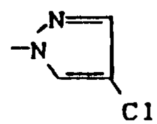 | H | H | H | H | 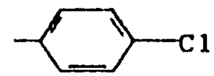 | 178° C |
| 268 | 4-$OCF_3$ | H | O | 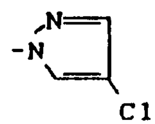 | H | H | H | H | 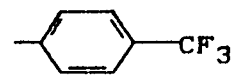 | 174° C |
| 269 | 4-$OCF_3$ | H | O | 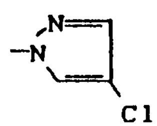 | H | H | H | H | 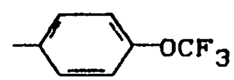 | 170° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 270 | 4-$CF_3$ | H | O | 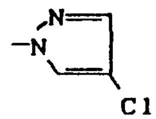 | H | H | H | H | 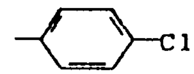 | 233° C |
| 271 | 4-$CF_3$ | H | O | 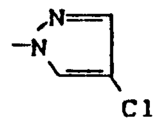 | H | H | H | H | 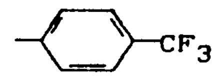 | 107° C |
| 272 | 4-$CF_3$ | H | O | 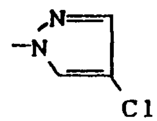 | H | H | H | H | 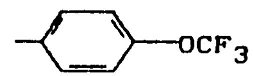 | 224° C |
| 273 | 4-Cl | H | O | 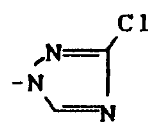 | H | H | H | H | 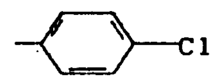 | >250° C |
| 274 | 4-Cl | H | O | 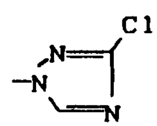 | H | H | H | H | 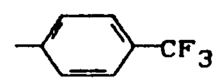 | 203° C |
| 274a | 4-Cl | H | O | 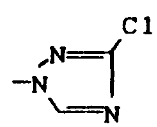 | H | H | H | H | 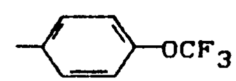 | |

| Bsp.Nr. | Y | Z | X | $R^1$ | $R^2$ | $R^3$ | $R^4$ | $R^5$ | $R^6$ | Fp |
|---|---|---|---|---|---|---|---|---|---|---|
| 275 | 4-Cl | H | O | 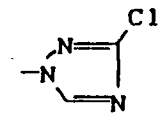 | H | H | H | H | 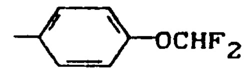 | 221° C |
| 276 | 4-$OCHF_2$ | H | O | 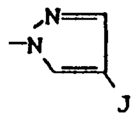 | H | H | H | H | 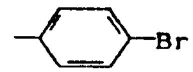 | 184° C |
| 277 | 4-$OCHF_2$ | H | O | 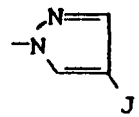 | H | H | H | H | 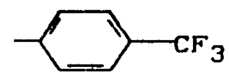 | |
| 277a | 4-$OCHF_2$ | H | O | 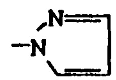 | H | H | H | H | 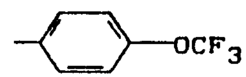 | |
| 277b | 4-F | H | O | 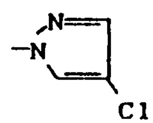 | H | H | H | H | 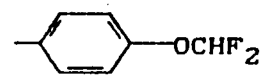 | |
| 277c | 4-Cl | H | O | 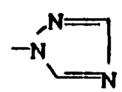 | H | H | H | H | 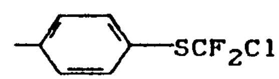 | |
| 277d | 4-F | H | O | 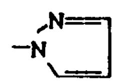 | H | H | H | H | 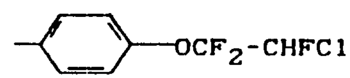 | |
| 277e | 3,4 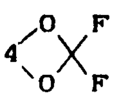 | H | O | 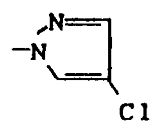 | H | H | H | H | 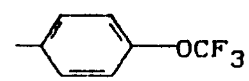 | |

Beispiel 278

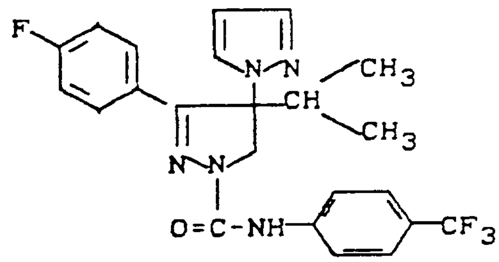

5 g (0,012 Mol) 3-(4-Fluorphenyl)-4-(1H-pyrazol-1-yl)-4,5-dihydro-1-pyrazolcarbonsäure-(4-trifluormethylanilid) werden in 80 ml Tetrahydrofuran (wasserfrei) gelöst und unter Argonatmosphäre und Rühren bei -15 °C 16,5 ml (0,026 Mol) einer 15-prozentigen Butyllithium-Lösung in Hexan zugesetzt. Es wird eine Stunde bei -15 °C nachgerührt und dann werden bei -15 °C 5 ml (0,05) Mol 2-Jodpropan zugesetzt. Man erwärmt dann auf Raumtemperatur (ca. 20 °C) und versetzt nach 30 Minuten die Reaktionsmischung mit 50 ml Wasser. Anschließend wird mit 100 ml Ether extrahiert, die Etherphase getrocknet und das Lösungsmittel im Vakuum abdestilliert. Der ölige Rückstand wird mit Hexan/Aceton (7:3) über Kieselgel chromatographiert. Man erhält 3,3 g 3-(4-Fluorphenyl)-4-(1H-pyrazol-1-yl)-4-isopropyl-4,5-dihydro-1-pyrazolcarbonsäure-(4-trifluormethylanilid) als farblosen Schaum. Fp. 125 °C

$^1$H-NMR ($CDCl_3$, TMS, ppm)

8,236 (1H,s), 7,66-7,0 (10H, m), 6,388 (1H,t), 4,535-4,378 (2H, q), 3,3-3,2(1H,m), 1,23 (3H, d) und 1,91 (3H, d).

In analoger Weise sind herstellbar:

**Allgemeine Formel**

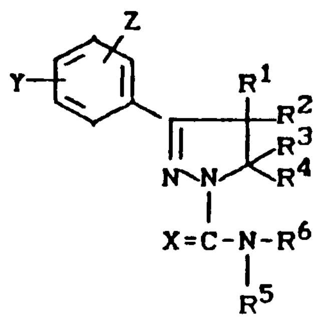

(I)

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 279 | 4-F | H | O | 1H-Pyrazol-1-yl | $CH_3$ | H | H | H | 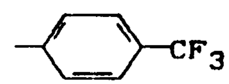 | 182-184° C |
| 280 | 4-Cl | H | O | 1H-Pyrazol-1-yl | $CH_3$ | H | H | H | 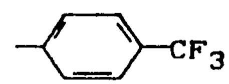 | |
| 281 | $4-OCHF_2$ | H | O | 1H-Pyrazol-1-yl | $CH_3$ | H | H | H | 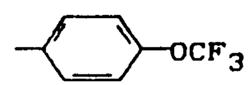 | |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 282 | 4-F | H | O | 1H-Pyrazol-1-yl | $COOCH_3$ | H | H | H | 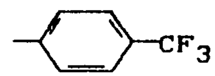 | |
| 283 | 4-$OCHF_2$ | H | O | 1H-Pyrazol-1-yl | $COOCH_3$ | H | H | H | 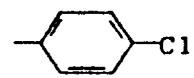 | |
| 284 | 4-F | H | O | 1,2,4-1H-Triazol-1-yl | $CH_3$ | H | H | H | 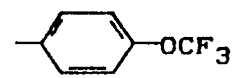 | |
| 285 | 4-F | H | O | 1,2,4-1H-Triazol-1-yl | 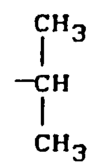 | H | H | H | 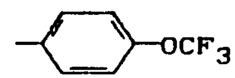 | 135° C |
| 286 | 4-Cl | H | O | 4-Chlor-1H-Pyrazol-1-yl | $-CH_3$ | H | H | H | 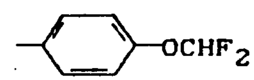 | 180-182° C |

| Bsp. Nr. | Y | Z | X | $R^1$ | $R^2$ | $R^3$ | $R^4$ | $R^5$ | $R^6$ | Fp |
|---|---|---|---|---|---|---|---|---|---|---|
| 287 | 4-F | H | O | 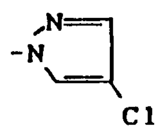 | $-COOCH_3$ | H | H | H | 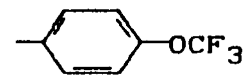 | 169° C |
| 288 | 4-F | H | O | 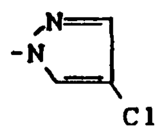 | $-Si(CH_3)_3$ | H | H | H | 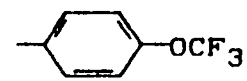 | Harz |
| 288a | $4-OCHF_2$ | H | O | 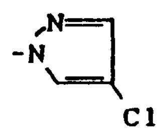 | $-Si(CH_3)_3$ | H | H | H | 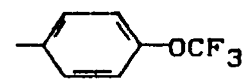 | Harz |

Herstellung von Ausgangsverbindungen

Beispiel 1A

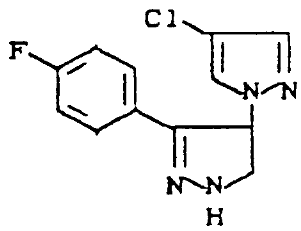

6 g (0,024 Mol) 4'-Fluor-2-(1''H-4''-chlorpyrazol-1''-yl)-acrylophenon werden in 60 ml Ethanol gelöst und unter Rühren 3 ml Hydrazinhydrat hinzugegeben. Man hält dabei die Temperatur unter Kühlung mit einem Wasserbad bei 40-45 °C. Es wird noch 30 Minuten nachgerührt und anschließend das Ethanol im Vakuum abdestilliert. Der Rückstand wird mit 150 ml Methylenchlorid versetzt, geschüttelt und dann die Methylenchloridphase abgetrennt und über Magnesiumsulfat getrocknet. Anschließend wird das Lösungsmittel im Vakuum abdestilliert und der ölige Rückstand mit Toluol/Essigsäureethylester (10:1) zur Kristallisation gebracht. Man erhält 3,5 g 3-(4'-Fluorphenyl)-4-(1''H-4''-chlorpyrazol-1''-yl)-4,5-dihydropyrazol als hellgelbe Kristalle mit dem Schmelzpunkt 127-128 °C.

In analoger Weise erhält man folgende Pyrazoline:

**Allgemeine Formel**

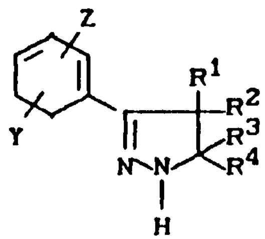

| Bsp. Nr. | Y | Z | $R^1$ | $R^2$ | $R^3$ | $R^4$ | Physikalische Konstanten |
|---|---|---|---|---|---|---|---|
| 2A | 4-$OCHF_2$ | H | 1-H-Pyrazol-1-yl | H | H | H | Öl |
| 3A | 4-$OCHF_2$ | H | 1,2,4-1H-Triazol-1-yl | H | H | H | Öl |
| 4A | 4-Cl | H | 1H-Pyrazol-1-yl | H | H | H | Öl, $^1$H-NMR($CDCl_3$): 7,15-7,55 (6H,m) 6,22(1H,s), 6,0(1H,q), 3,73-3,94 (2H,m) |
| 5A | 4-$OCHF_2$ | H | 1H-4-Chlor-pyrazol-1-yl | H | H | H | Öl, $^1$H-NMR($CDCl_3$): 7,61 (2H,d) 7,08(2H,d), 7,33 (1H,s), 7,44(1H,s), 6,51 (1H,t) 5,92 (1H,q), 3,72-3,9(2H,m) |
| 6A | 4-Cl | H | 1H-4-Chlor-pyrazol-1-yl | H | H | H | Fp: 177° C |

| Bsp. Nr. | Y | Z | $R^1$ | $R^2$ | $R^3$ | $R^4$ | Physikalische Konstanten |
|---|---|---|---|---|---|---|---|
| 7A | 4-F | H | 1,2,4-1H-Triazol-1-yl | H | H | H | Fp: 116-118° C |
| 8A | 4-Cl | H | 1,2,4-1H-Triazol-1-yl | H | H | H | Fp: 175° C |
| 9A | 4-F | H | 1-H-Pyrazol-1-yl | H | H | H | Fp: 130° C |
| 10A | 4-$OCF_3$ | H | 1,2,4-1H-Triazol-1-yl | H | H | H | Öl |
| 11A | 4-Cl | H | 3,5-Dimethyl-1H-pyrazol-1-yl | H | H | H | Öl, $^1$H-NMR($CDCl_3$, TMS): 7,20-7,40(4H,m) 6,04-5,98(1H,q); 4,19(1H,s); 4,03-3,69(2H,m) 2,20(3H,s); 2,18 (3H,s) |
| 12A | 4-F | H | 3,5-Dimethyl-1H-pyrazol-1-yl | H | H | H | Öl |
| 13A | H | H | 1H-Pyrazol-1-yl | H | H | H | |
| 14A | H | H | 1,2,4-1H-Triazol-1-yl | H | H | H | |
| 15A | 4-Br | H | 1H-Pyrazol-1-yl | H | H | H | Schmp. 143° C |
| 16A | 4-$CF_3$ | H | 1H-Pyrazol-1-yl | H | H | H | |
| 17A | 4-$CF_3$ | H | 4-Chlor-1H-pyrazol-1-yl | H | H | H | |
| 18A | 4-$CF_3$ | H | 1,2,4-1H-Triazol-1-yl | H | H | H | |
| 19A | 4-$CF_3$ | H | 1H-Pyrazol-1-yl | H | H | H | |

| Bsp. Nr. | Y | Z | $R^1$ | $R^2$ | $R^3$ | $R^4$ | Physikalische Konstanten |
|---|---|---|---|---|---|---|---|
| 20A | 4-Br | H | 1,2,4-1H-Triazol-1-yl | H | H | H | Schmp. 163° C |
| 21A | 4-Br | H | 1H-4-Chlor-pyrazol-1-yl | H | H | H | Schmp. 189° C |
| 22A | 4-F | H | 1,2,3,4-2H-Tetrazol-2-yl | H | H | H | |
| 23A | $4-OCH_2CF_3$ | H | 1H-Pyrazol-1-yl | H | H | H | |
| 24A | 4-Cl | H | 1,2,4-4H-Triazol-1-yl | H | H | H | |
| 25A | 4-Cl | H | 1,2,4-1H-Triazol-1-yl | H | $CH_3$ | H | |
| 26A | 4-F | H | 1H-Pyrazol-1-yl | H | $CH_3$ | $CH_3$ | |
| 27A | $4-OCF_2-CHFCl$ | H | 1H-4-Chlor-pyrazol-1-yl | H | H | H | |
| 28A | 4-Cl | H | 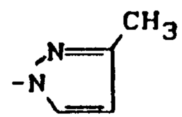 | H | H | H | Öl |
| 29A | $4-OCHF_2$ | H | 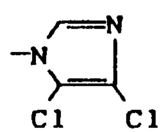 | H | H | H | Öl |

| Bsp. Nr. | Y | Z | $R^1$ | $R^2$ | $R^3$ | $R^4$ | Physikalische Konstanten |
|---|---|---|---|---|---|---|---|
| 30A | 4-Br | H | 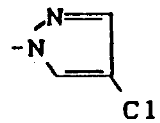 | H | H | H | |
| 31A | 4-$OCHF_2$ | H | 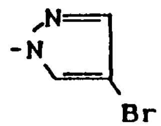 | H | H | H | Schmp. 219° C |
| 32A | H | H | 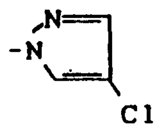 | H | H | H | Schmp. 139° C |
| 33A | 4-Cl | H | 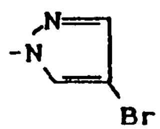 | H | H | H | |
| 34A | H | H | 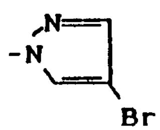 | H | H | H | |

| Bsp. Nr. | Y | Z | $R^1$ | $R^2$ | $R^3$ | $R^4$ | Physikalische Konstanten |
|---|---|---|---|---|---|---|---|
| 35A | 4-$OCH_3$ | H | 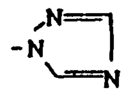 | H | H | H | Öl |
| 36A | 4-$OCH_3$ | H | 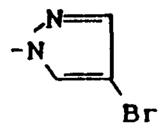 | H | H | H | Öl |
| 37A | 4-$OCH_3$ | H | 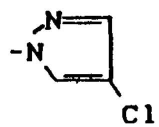 | H | H | H | Öl |
| 38A | 4-$OCH_3$ | H | 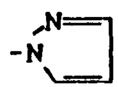 | H | H | H | Öl |
| 39A | 4-$OC_2H_5$ | H | 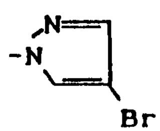 | H | H | H | |

| Bsp. Nr. | Y | Z | $R^1$ | $R^2$ | $R^3$ | $R^4$ | Physikalische Konstanten |
|---|---|---|---|---|---|---|---|
| 40A | 3-$CH_3$ | H | 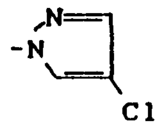 | H | H | H | Öl |
| 41A | 4-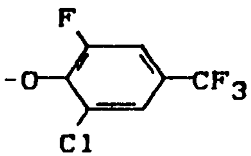 | H | 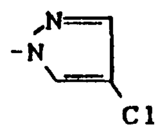 | H | H | H | Öl |
| 42A | 4-Cl | H | 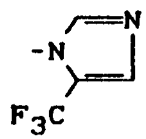 | H | H | H | |
| 43A | 4-Cl | H | 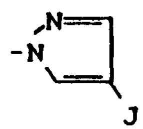 | H | H | H | |
| 44A | 4-Br | H | 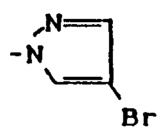 | H | H | H | Schmp. 189° C |

| Bsp. Nr. | Y | Z | $R^1$ | $R^2$ | $R^3$ | $R^4$ | Physikalische Konstanten |
| --- | --- | --- | --- | --- | --- | --- | --- |
| 45A | 3,4 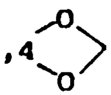 | H | 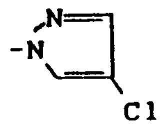 | H | H | H | Öl |
| 46A | 3,4 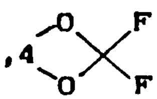 | H | 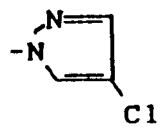 | H | H | H | |
| 47A | 3,4 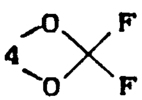 | H | 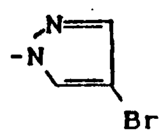 | H | H | H | Öl |
| 48A | 4-F | H | 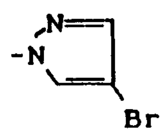 | H | H | H | Öl |
| 49A | 3,4-$OCH_3$ | H | 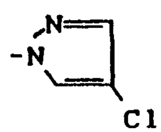 | H | H | H | Öl |

| Bsp. Nr. | Y | Z | $R^1$ | $R^2$ | $R^3$ | $R^4$ | Physikalische Konstanten |
|---|---|---|---|---|---|---|---|
| 50A | 3-Cl | H | 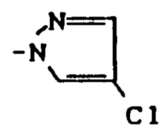 | H | H | H | |
| 51A | 4-$OCF_3$ | H | 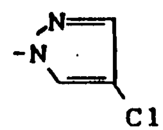 | H | H | H | |
| 52A | 4-Cl | H | 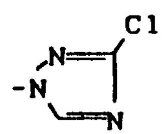 | H | H | H | |
| 53A | 4-$OCHF_2$ | H | 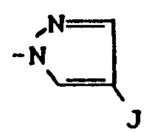 | H | H | H | |

Beispiel 54 A

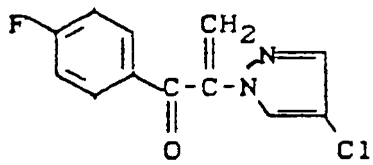

23,9 g (0,1 Mol) α-(1H-4-Chlorpyrazol-1-yl)-4'-fluoracetophenon werden in 30 ml Methanol bei 20 bis 30 °C suspendiert. Anschließend gibt man unter Rühren 40 ml 37-prozentige Formalinlösung, dann 5 ml Piperidin und anschließend 5,5 ml Eisessig hinzu, dabei steigt die Temperatur auf 35 bis 40 °C. Die Reaktionsmischung wird dann 4 bis 5 Stunden bei Raumtemperatur (ca. 20 °C) gerührt bis nach Dünnschichtchromatographie-Kontrolle kein Ausgangsmaterial mehr vorhanden ist. Die Reaktionsmischung wird dann mit Ether auf 300 ml verdünnt, die Etherphase abgetrennt und über Magnesiumsulfat getrocknet. Zur Reinigung wird die getrocknete Etherphase über Kieselgel filtriert. Anschließend wird der Ether im Vakuum abdestilliert. Man erhält 16 g eines gelben Öls. Das so erhaltene 4'-Fluor-2-(1"H-4"-Chlorpyrazol-1"-yl) acrylophenon wird ohne weitere Reinigung weiter umgesetzt.

$^{1}$H-NMR: ($CDCl_3$, TMS, ppm)

7,85-7,93 (2H,m); 7,09-7,2(2H,m); 7,60 (1H,s); 7,84 (1H,s); 5,56 (1H,s), 6,34 (1H,s).

In analoger Weise sind die folgenden Derivate erhältlich:

**Allgemeine Formel:**

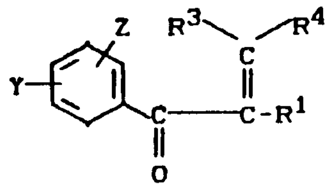

| Bsp. Nr. | Y | Z | $R^1$ | $R^3$ | $R^4$ | Physikalische Konstanten |
|---|---|---|---|---|---|---|
| 55A | 4-$OCHF_2$ | H | 1,2,4-1H-Triazol-1-yl | H | H | Öl, $^1H$-NMR($CDCl_3$ 8,68 (1H,s); 8,06 (1H,s), 7,915 (2H,d); 7,25(2H,d) 6,63 (1H,t), 6,68(1H,s); 5,8 (1H,s) |
| 56A | 4-$OCHF_2$ | H | 1H-Pyrazol-1-yl | H | H | Öl |
| 57A | 4-Cl | H | 1H-Pyrazol-1-yl | H | H | Öl |
| 58A | 4-Cl | H | 4-Chlor-1H-pyrazol-1-yl | H | H | Öl, ($^1H$-NMR ($CDCl_3$) 6,35 (1H,s), 5,58 (1H,s) |
| 59A | 4-$OCHF_2$ | H | 4-Chlor-1-H-pyrazol-1-yl | H | H | Öl |
| 60A | 4-F | H | 1,2,4-1H-Triazol-1-yl | H | H | Öl, $^1H$-NMR ($CDCl_3$) 8,68 (1H,s), 8,05 (1H,s), 7,89-7,96 (2H,m), 7,24-,7,17 (2H,m); 6,67 (1H,s); 5,79 (1H,s) |

| Bsp. Nr. | Y | Z | $R^1$ | $R^3$ | $R^4$ | Physikalische Konstanten |
|---|---|---|---|---|---|---|
| 61A | 4-Cl | H | 1,2,4-1H-Triazol-1-yl | H | H | Fp: 122° C |
| 62A | 4-$OCF_3$ | H | 1,2,4-1H-Triazol-1-yl | H | H | Öl |
| 63A | 4-Cl | H | 3,5-Dimethyl-1H-pyrazol-1-yl | H | H | Öl |
| 64A | 4-F | H | 3,5-Dimethyl-1H-pyrazol-1-yl | H | H | Öl |
| 65A | 4-F | H | 1H-Pyrazol-1-yl | H | H | Öl |
| 66A | H | H | 1H-Pyrazol-1-yl | H | H | Öl |
| 67A | H | H | 1,2,4-1H-Triazol-1-yl | H | H | Öl |
| 68A | 4-Br | H | 1H-Pyrazol-1-yl | H | H | Öl |
| 69A | 4-$CF_3$ | H | 1H-Pyrazol-1-yl | H | H | Öl |
| 70A | 4-$CF_3$ | H | 4-Chlor-1H-pyrazol-1-yl | H | H | Öl |
| 71A | 4-$CF_3$ | H | 1,2,4-1H-Triazol-1-yl | H | H | Öl |
| 72A | 4-$CH_3$ | H | 1H-Pyrazol-1-yl | H | H | Öl |
| 73A | 4-$OCH_2CF_3$ | H | 1H-Pyrazol-1-yl | H | H | Öl |
| 74A | 4-Br | H | 1,2,4-1H-Triazol-1-yl | H | H | Öl |

| Bsp. Nr. | Y | Z | $R^1$ | $R^2$ | $R^3$ | $R^4$ | Physikalische Konstanten |
|---|---|---|---|---|---|---|---|
| 75A | 4-Br | H | 1H-4-Chlor-pyrazol-1-yl | | H | H | |
| 76A | 4-F | H | 1,2,3,4-2H-Tetrazol-1-yl | | H | H | Öl |
| 77A | 4-Cl | H | 1,2,4-1H-Triazol-1-yl | | $CH_3$ | H | Öl |
| 78A | 4-F | H | 1H-Pyrazol-1-yl | | $CH_3$ | $CH_3$ | Öl |
| 79A | H | H | 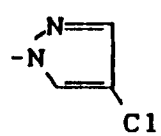 | H | H | H | Öl |
| 80A | 4-Cl | H | 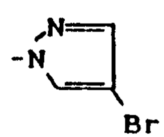 | H | H | H | Öl |
| 81A | H | H | 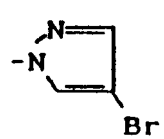 | H | H | H | Öl |

| Bsp. Nr. | Y | Z | $R^1$ | $R^2$ | $R^3$ | $R^4$ | Physikalische Konstanten |
|---|---|---|---|---|---|---|---|
| 82A | 4-$OCH_3$ | H | 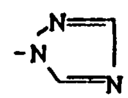 | H | H | H | Öl |
| 83A | 4-$OCH_3$ | H | 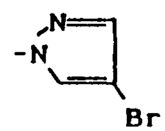 | H | H | H | Öl |
| 84A | 4-$OCH_3$ | H | 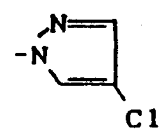 | H | H | H | Öl |
| 85A | 4-$OCH_3$ | H | 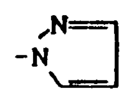 | H | H | H | Öl |
| 86A | 4-$OCH_3$ | H | 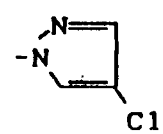 | H | H | H | Öl |
| 87A | 3-$CH_3$ | H | 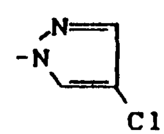 | H | H | H | Öl |

| Bsp. Nr. | Y | Z | $R^1$ | $R^2$ | $R^3$ | $R^4$ | Physikalische Konstanten |
|---|---|---|---|---|---|---|---|
| 88A | 4-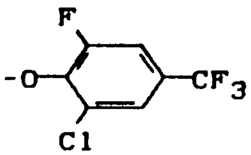 | H | 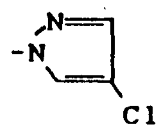 | H | H | H | Öl |
| 89A | 4-Cl | H | 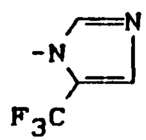 | H | H | H | Öl |
| 90A | 4-Cl | H | 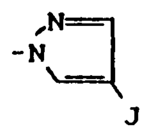 | H | H | H | Öl |
| 91A | 4-Br | H | 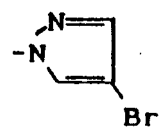 | H | H | H | Öl |
| 92A | 3,4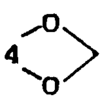 | | 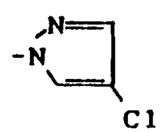 | H | H | H | Öl |

| Bsp. Nr. | Y | Z | $R^1$ | $R^2$ | $R^3$ | $R^4$ | Physikalische Konstanten |
|---|---|---|---|---|---|---|---|
| 93A | 3,4 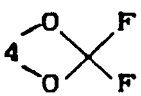 | | 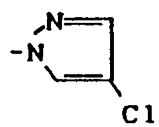 | H | H | H | Öl |
| 94A | 3,4 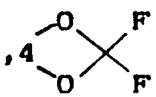 | | 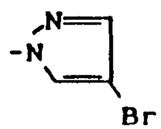 | H | H | H | Öl |
| 95A | 4-F | H | 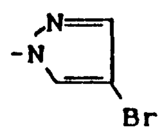 | H | H | H | Öl |
| 96A | 3,4-$OCH_3$ | H | 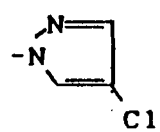 | H | H | H | Öl |
| 97A | 3-Cl | H | 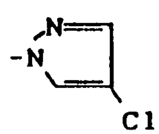 | H | H | H | Öl |

| Bsp. Nr. | Y | Z | $R^1$ | $R^2$ | $R^3$ | $R^4$ | Physikalische Konstanten |
|---|---|---|---|---|---|---|---|
| 98A | 4-$OCF_3$ | H | 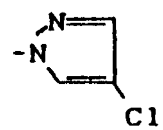 | H | H | H | Öl |
| 99A | 4-Cl | H | 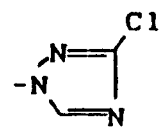 | H | H | H | Öl |
| $100A_1$ | 4-$OCHF_2$ | H | 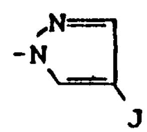 | H | H | H | Öl |
| $100A_2$ | 4-$OCHF_2$ | H | 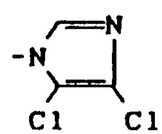 | H | H | H | Öl |

Beispiel 101 A

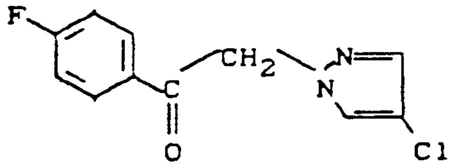

51,3 g (0,5 Mol) 4-Chlorpyrazol werden in 700 ml Acetonitril gelöst und mit 150 g Kaliumcarbonat versetzt. Unter Rühren gibt man hierzu 100 g α-Brom-4-fluor-acetophenon und erhitzt die Mischung unterRühren vier Stunden zum Rückfluß. Anschließend wird im Vakuum die Hälfte des Lösungsmittels abdestilliert und der Rückstand mit 500 ml Ether versetzt und dann in 500 ml Wasser gegossen.

Die Etherphase wird abgetrennt, über Magnesiumsulfat getrocknet und das Lösungsmittel im Vakuum abdestilliert. Man erhält 102 g α-(1H-4-Chlorpyrazol-1-yl)-4'-fluoracetophenon als kristallinen Rückstnd mit dem Schmelzpunkt (Fp) 136 ° C.

In analoger Weise sind die folgenden Verbindungen erhältlich:

| Bsp. Nr. | Formel | Schmelzpunkt |
|---|---|---|
| 102A | 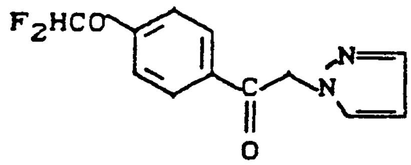 | 122-124° C |
| 103A | 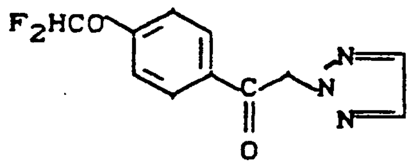 | 100-102° C |
| 104A | 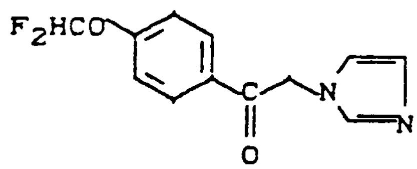 | |
| 105A | 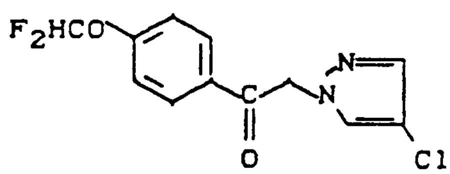 | |
| 106A | 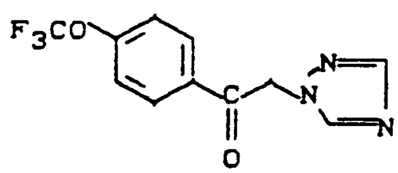 | |
| 107A | 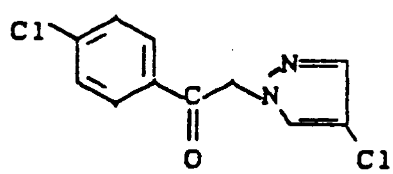 | 135-136° C |
| 108A | 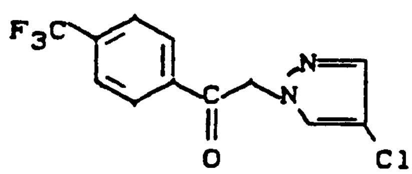 | |
| 109A | 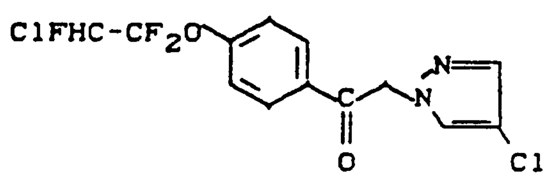 | |

| Bsp. Nr. | Formel | Schmelzpunkt |
|---|---|---|
| 110A | 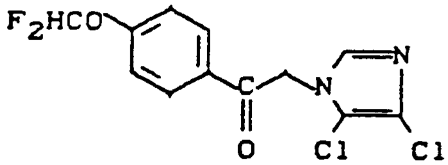 | |
| 111A | 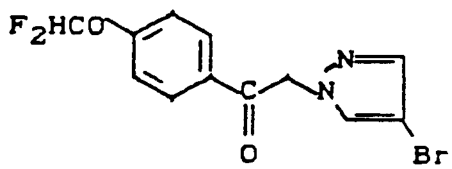 | 105° C |
| 112A | 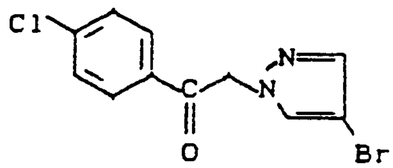 | 134° C |
| 113A | 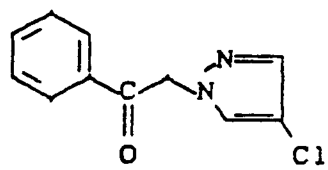 | 148° C |
| 114A | 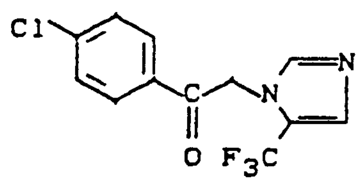 | |
| 115A | 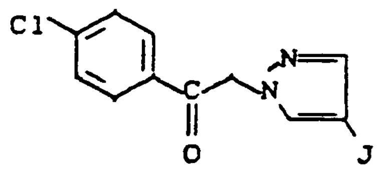 | |
| 116A | 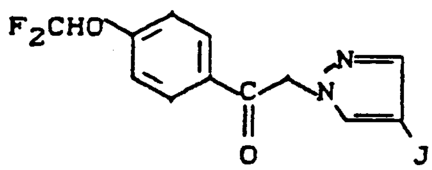 | |
| 117A | 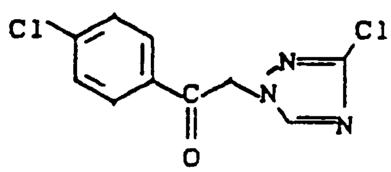 | |

| Bsp. Nr. | Formel | Schmelzpunkt |
|---|---|---|
| 118A | 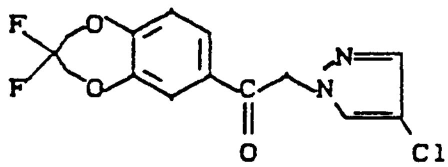 | |

## Anwendungsbeispiele

In den folgenden Anwendungsbeispielen wurden die nachstehend aufgeführten Verbindungen als Vergleichssubstanzen eingesetzt.

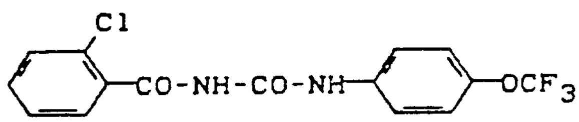

Triflumuron = 2-Chlor-N[[[4-(trifluormethoxy)-phenyl]-amino]-carbonyl]-benzamid
(bekannt aus: DE-A 2 601 780)

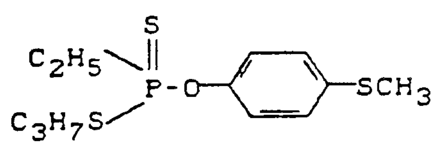

Sulprofos = O-(Ethyl-O-(4-methylthio)-phenyl)-S-propyldithiophosphat
(bekannt aus: DE-A 2 111 414)

## Beispiel A

Plutella-Test

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1, 9, 36, 43, 45, 46, 60, 67, 102

## Beispiel B

Heliothis virescens-Test

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) werden durch Tauchen in die Wirkstoffzubereitung der gewünschten Konzentration behandelt und mit der Tabakknospenraupe (Heliothis virescens) besetzt, solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 9, 23, 24, 27, 28, 29, 55, 60, 73, 74, 87, 88, 93, 94

Beispiel C

$LD_{100}$-Test

| | |
|---|---|
| Testtiere: | Sitophilus granarius |
| Lösungsmittel: | 35 Gewichtsteile Ethylenglykolmonomethylether |
| | 35 Gewichtsteile Nonylphenolpolyglykolether |

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierschalen (Durchmesser 9,5 cm) pipettiert, die sich in Petrischalen entsprechender Größe befinden. Nach Trocknung der Filterscheibe werden ca. 30 Testtiere in die Petrischalen überführt und abgedeckt.

Der Zustand der Testtiere wird 3 Tage nach Ansetzen der Versuche kontrolliert. Es wird diejenige Zeit ermittelt, die für eine 100%ige knock-down Wirkung erforderlich ist. Wird die $LD_{100}$ nach 6 Stunden nicht erreicht, wird der Prozentsatz der knock-down gegangenen Testtiere festgestellt.

Bei diesem Test zeigen z.B. die folgenden Verbindungen, der Herstellungsbeispiele eine stark ausgeprägte Wirksamkeit: 1, 4, 9, 18, 20, 28, 29, 42, 43, 44, 45, 46, 53, 60, 62, 63, 64, 67, 69, 70, 73, 74, 77, 78, 79, 80, 82, 83, 86, 93, 94, 122, 123, 140, 145

Beispiel D

Test mit Lucilia cuprina resistent-Larven

| | |
|---|---|
| Emulgator: | 35 Gewichtsteile Ethylenglykolmonomethylether |
| | 35 Gewichtsteile Nonylphenolpolyglykolether |

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigen z.B. die folgenden Verbindungen der Herstellungsbeispiele eine stark ausgeprägte Wirksamkeit: 1, 4, 9, 10, 16, 17, 18, 20, 28, 36, 37, 42, 45, 46, 49, 53, 55, 60, 63, 64, 66, 67, 68, 69, 73, 74, 83, 93, 94, 104, 122, 123, 140, 145.

Beispiel E

$LD_{100}$-Test

| | |
|---|---|
| Testtiere: | Blattella germanica |
| Lösungsmittel: | 35 Gewichtsteile Ethylenglykolmonomethylether |
| | 35 Gewichtsteile Nonylphenolpolyglykolether |

Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration.

2 ml dieser Wirkstoffzubereitung werden auf Filterpapierschalen (0 9,5 cm) pipettiert, die sich in Petrischalen entsprechender Größe befinden. Nach Trocknung der Filterscheiben werden 5 Testtiere in die

Petrischalen überführt und abgedeckt.

Der Zustand der Testtiere wird 3 Tage nach Ansetzen der Versuche kontrolliert. Es wird diejenige Zeit ermittelt, die für eine 100 %ige knock down-Wirkung erforderlich ist. Wird die $LD_{100}$ nach 6 Stunden nicht erreicht, wird der Prozentsatz der knock down gegangenen Testiere festgestellt.

Bei diesem Test zeigen z.B. die Verbindungen der Herstellungsbeispiele 4, 20, 62, 64, 67, 74, 122, 123, 140 eine stark ausgeprägte Wirksamkeit.

**Patentansprüche**

**1.** Substituierte Pyrazolinderivate der allgemeinen Formel (I)

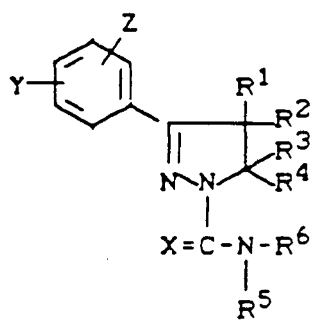

(I)

in welcher

- $R^1$ für einen gegebenenfalls durch Halogen, Alkyl($C_1$-$C_6$), CN, $NO_2$, Alkoxy($C_1$-$C_6$)carbonyl, Alkyl($C_1$-$C_4$) thio, Alkoxy($C_1$-$C_6$), Halogenalkyl($C_1$-$C_4$), Halogenalkyl($C_1$-$C_4$)thio, Halogenalkoxy ($C_1$-$C_4$), Dialkyl($C_1$-$C_4$)-amino, substituierten 1H-Pyrrolyl-(1)-, 1H-Pyrazolyl-(1)-, 1H-Imidazolyl-(1)-, 2H-1,2,3-Triazolyl-(2)-, 1H-1,2,3,-Triazolyl-(1)-, 1H-1,2,4-Triazolyl-(1)-, 4H-1,2,4-Triazolyl-(4)-, 2H-Tetrazolyl-(2)- oder 1H-Tetrazolyl-(2)-Rest steht,
- $R^2$ für Wasserstoff, Alkyl($C_1$-$C_6$), gegebenenfalls durch Halogen, Halogenalkyl($C_1$-$C_4$) substituiertes Cycloalkyl($C_3$-$C_7$); Halogenalkyl($C_1$-$C_4$)thio, Alkoxy ($C_1$-$C_6$)carbonyl oder Trialkyl($C_1$-$C_6$)-silyl steht,
- $R^3$ für Wasserstoff oder Alkyl ($C_1$-$C_6$) steht,
- $R^4$ für Wasserstoff oder Alkyl($C_1$-$C_6$) steht,
- $R^5$ für Wasserstoff, Alkyl($C_1$-$C_6$), Phenyl oder Alkyl ($C_1$-$C_4$)thio steht,
- $R^6$ für gegebenenfalls durch Halogen, Halogenalkyl($C_1$-$C_4$), Halogenalkoxy($C_1$-$C_4$) substituiertes Alkyl($C_1$-$C_6$),
  für gegebenenfalls durch Halogen, Halogenalkyl($C_1$-$C_4$) Halogenalkoxy($C_1$-$C_4$) substituiertes Cycloalkyl ($C_3$-$C_7$) oder für den Rest

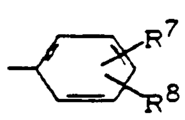

steht,

wobei $R^7$ und $R^8$ gleich oder verschieden sein können und für Wasserstoff, Halogen, Alkyl-($C_1$-$C_6$), Nitro, Cyano, Halogenalkyl($C_1$-$C_6$), Alkoxy($C_1$-$C_6$), Halogenalkoxy ($C_1$-$C_6$), Alkyl($C_1$-$C_6$)thio, Halogenalkyl($C_1$-$C_6$)thio, gegebenenfalls durch Halogen, Halogenalkyl($C_1$-$C_6$), Alkoxy-($C_1$-$C_6$), Alkyl($C_1$-$C_6$) substituiertes Phenoxy oder Phenylthio, gegebenenfalls durch Halogen, Alkoxy($C_1$-$C_4$), Halogenalkyl($C_1$-$C_4$) substituiertes Mono- oder Dialkylamino mit jeweils 1 bis 6 Kohlenstoffatomen im Alkylrest, gegebenenfalls durch Alkyl ($C_1$-$C_6$), Alkoxy($C_1$-$C_4$), Halogen, Alkyl($C_1$-$C_4$)thio substituiertes Cycloalkyl($C_3$-$C_7$), Alkoxy($C_1$-$C_6$)carbonyl, Alkenyl($C_2$-$C_6$)-oxy, Alkinyl($C_2$-$C_6$), Alkyl(C1-$C_4$)thionyl, Alkyl($C_1$-$C_4$)sulfonyl, Halogenalkyl($C_1$-$C_4$)-thionyl, Halogenalkyl($C_1$-$C_4$) sulfonyl stehen oder wobei

$R^7$ und $R^8$ zusammen für einen der folgenden bivalenten Reste stehen:

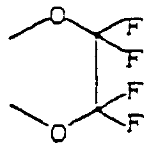 , 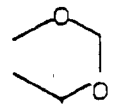 , 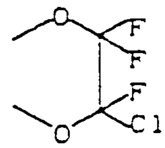 , 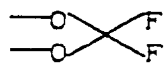 ,

X für Sauerstoff oder Schwefel steht und

Y und Z gleich oder verschieden sein können und für Wasserstoff, Alkyl($C_1$-$C_6$), Halogen, Halogenalkyl($C_1$-$C_6$), Alkoxy($C_1$-$C_6$), Alkyl($C_1$-$C_6$)thio, Halogenalkoxy($C_1$-$C_4$), Halogenalkyl($C_1$-$C_4$)thio, Alkoxy($C_1$-$C_4$)carbonyl, gegebenenfalls durch Halogen, Alkyl($C_1$-$C_4$), Alkoxy($C_1$-$C_4$, Halogenalkyl ($C_1$-$C_4$) substituiertes Phenoxy oder Phenylthio, Alkenyl($C_2$-$C_6$)oxy, Alkinyl($C_2$-$C_6$), Alkyl($C_1$-$C_4$)thionyl, Alkyl($C_1$-$C_4$)sulfonyl, Halogenalkyl($C_1$-$C_4$)thionyl, Halogenalkyl($C_1$-$C_4$)sulfonyl, gegebenenfalls durch Halogen, Alkoxy($C_1$-$C_4$), Halogenalkyl($C_1$-$C_4$)substituiertes Mono- oder Dialkyl($C_1$-$C_6$)amino, Nitro, Cyano stehen oder wobei

Y und Z zusammen für gegebenenfalls durch Fluor und/oder Chlor substituiertes 3,4-Methylendioxy oder 3,4-Ethylendioxy stehen.

**2.** Substituierte Pyrazolinderviate der Formel (I) gemäß Anspruch 1, in welcher

$R^1$ für einen gegebenenfalls durch Fluor, Chlor, Brom, Jod, Alkyl($C_1$-$C_4$), CN, $NO_2$, Alkoxy($C_1$-$C_4$)carbonyl, Alkyl($C_1$-$C_3$)thio, Alkoxy($C_1$-$C_4$), Halogenalkyl ($C_1$-$C_3$), Halogenalkyl($C_1$-$C_3$)thio oder Halogenalkoxy($C_1$-$C_4$)carbonyl substituierten 1H-Pyrrolyl-(1)-, 1H-Pyrazolyl-(1)-, 1H-Imidazolyl-(1)-, 2H-1,2,3,-Triazolyl-(2)-, 1H-1,2,3-Triazolyl-(1)-, 1H-1,2,4-Triazolyl-(1)-, 4H-1,2,4-Triazolyl-(4)-, 2H-Tetrazolyl-(2)-oder 1H-Tetrazolyl-(1)-Rest steht,

$R^2$ für Wasserstoff, Alkyl($C_1$-$C_4$), gegebenenfalls durch Fluor, Chlor, Brom, Halogenalkyl($C_1$-$C_3$)-substituiertes Cycloalkyl ($C_3$-$C_6$), Halogenalkyl($C_1$-$C_3$), Halogenalkyl($C_1$-$C_3$)thio, Alkoxy($C_1$-$C_4$)carbonyl oder Trialkyl($C_1$-$C_4$)silyl steht,

$R^3$ für Wasserstoff oder Alkyl($C_1$-$C_4$) steht,

$R^4$ für Wasserstoff oder Alkyl($C_1$-$C_4$) steht,

$R^5$ für Wasserstoff, Alkyl($C_1$-$C_4$), Phenyl oder Alkyl($C_1$-$C_3$)thio steht,

$R^6$ für gegebenenfalls durch Fluor, Chlor, Brom, Halogenalkyl($C_1$-$C_3$), Halogenalkoxy($C_1$-$C_3$)-substituiertes Alkyl($C_1$-$C_4$), für gegebenenfalls durch Fluor, Chlor, Brom, Halogenalkyl($C_1$-$C_3$), Halogenalkoxy($C_1$-$C_3$) substituiertes Cyloalkyl($C_3$-$C_6$) oder für den Rest

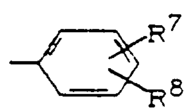

steht,

wobei $R^7$ und $R^8$ gleich oder verschieden sein können und für

Wasserstoff, Fluor, Chlor, Brom, Jod, Alkyl($C_1$-$C_4$), Nitro, Cyano, Halogenalkyl($C_1$-$C_3$), Alkoxy-($C_1$-$C_4$), Halogenalkoxy ($C_1$-$C_3$), Alkyl($C_1$-$C_3$)thio, Halogenalkyl($C_1$-$C_3$)thio, gegebenenfalls durch Fluor, Chlor, Brom, Halogenalkyl($C_1$-$C_3$), Alkoxy($C_1$-$C_3$), Alkyl($C_1$-$C_3$) substituiertes Phenoxy, gegebenenfalls durch Fluor, Chlor, Brom, Alkoxy($C_1$-$C_3$), Halogenalkyl($C_1$-$C_3$) substituierts Mono- oder Dialkylamino mit jeweils 1 bis 4 Kohlenstoffatomen im Alkylrest, gegebenenfalls durch Alkyl($C_1$-$C_3$), Alkoxy($C_1$-$C_3$), Fluor, Chlor, Brom, Alkyl($C_1$-$C_3$)thio substituiertes Cycloalkyl($C_3$-$C_6$) stehen oder wobei

$R^7$ und $R^8$ zusammen für eine der folgenden bivalenten Rest stehen:

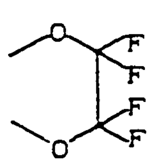 , 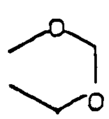 , 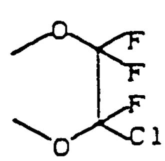 , 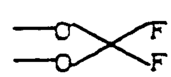 ,

X für Sauerstoff oder Schwefel steht und
Y und Z gleich oder verschieden sein können und für Wasserstoff, Alkyl($C_1$-$C_4$), Fluor, Chlor, Brom, Jod, Halogenalkyl($C_1$-$C_4$), Alkoxy($C_1$-$C_4$), Alkyl($C_1$-$C_4$) thio, Halogenalkoxy($C_1$-$C_3$), Halogenalkyl($C_1$-$C_3$)thio, Alkoxy($C_1$-$C_3$)carbonyl, gegebenenfalls durch Fluor, Chlor, Brom, Alkyl($C_1$-$C_3$), Alkoxy ($C_1$-$C_3$), Halogenalkyl($C_1$-$C_3$) substituiertes Phenoxy oder Phenylthio, Halogenalkoxy ($C_1$-$C_3$)carbonyl, Alkenyl($C_2$-$C_4$)oxy, Alkinyl($C_2$-$C_4$), Alkyl($C_1$-$C_3$) thionyl, Alkyl($C_1$-$C_3$)sulfonyl, Halogenalkyl($C_1$-$C_3$) thionyl, Halogenalkyl($C_1$-$C_3$)sulfonyl, gegebenenfalls durch Halogen, Alkoxy($C_1$-$C_3$), Halogenalkyl($C_1$-$C_3$) substituiertes Mono- oder Dialkyl($C_1$-$C_3$)amino, Nitro, Cyano stehen oder wobei
Y und Z zusammen für gegebenenfalls durch Fluor und/oder Chlor substituiertes 3,4-Methylendioxy oder 3,4-Ethylendioxy stehen.

**3.** Verfahren zur Herstellung von substituierten Pyrazolenderivaten der allgemeinen Formel (I)

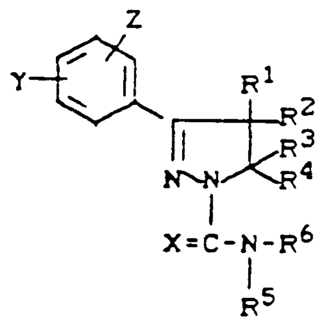

(I)

in welcher die Substituenten $R^1$, $R^2$, $R^3$, $R^4$, $R^5$, $R^6$, X, Y und Z die im Anspruch 1 angegebene Bedeutung haben, dadurch gekennzeichnet, daß man

(a) zum Erhalt von Pyrazolinderivaten der Formel (I), in welcher $R^5$ für Wasserstoff steht, Pyrazolinderivate der Formel (II)

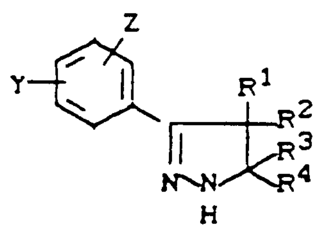

(II)

in welcher Y, Z, $R^1$, $R^2$, $R^3$ und $R^4$ die oben angegebene Bedeutung besitzen,
mit Isocyanaten bzw. Isothiocyanaten der Formel (III)

$X=C=N\text{-}R^6$ (III)

in welcher X und $R^6$ die oben angegebene Bedeutung haben,
gegebenenfalls in Gegenwart von Basen umsetzt
oder daß man

(b) zum Erhalt von Pyrazolinderivaten der Formel (I), in welcher $R^2$ für Alkyl($C_1$-$C_6$), Cycloalkyl($C_3$-$C_7$), Halogenalkyl($C_1$-$C_4$)thio, Alkoxy($C_1$-$C_6$)carbonyl oder Trialkyl($C_1$-$C_6$)silyl steht, Verbindungen der Formel (IV)

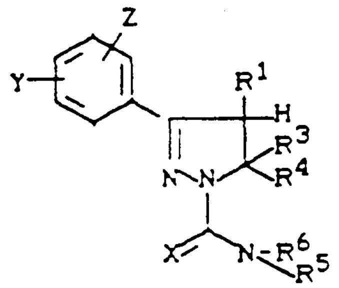

(IV)

mit Verbindungen der Formel (V)

Hal-$R^9$ (V)

in welcher

Hal für Halogen steht und

$R^9$ für Alkyl, Cycloalkyl, Halogenalkyl, Halogenalkylthio oder Alkoxycarbonyl steht,

in wasserfreiem Medium unter Zusatz einer starken Base umsetzt.

**4.** Substituierte Pyrazolinderivate der allgemeinen Formel (II)

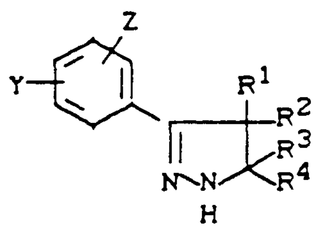

(II)

in welcher $R^1$, $R^2$, $R^3$, $R^4$, Y und Z die im Anspruch 1 angegebene Bedeutung haben.

**5.** Verbindungen der allgemeinen Formel (VI)

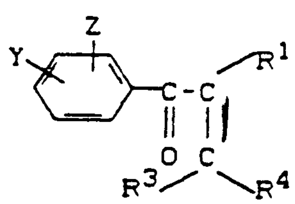

(VI)

in welcher $R^1$, $R^3$, $R^4$, Y und Z die im Anspruch 1 angegebene Bedeutung haben.

**6.** Insektizide und akarizide Mittel, gekennzeichnet durch einen Gehalt an mindestens einem substituierten Pyrazolinderivat der Formel (I).

**7.** Verfahren zur Bekämpfung von Insekten und Spinnentieren, dadurch gekennzeichnet, daß man substituierte Pyrazolinderivate der Formel (I) auf Insekten und/oder Spinnentiere und/oder ihren Lebensraum einwirken läßt.

**8.** Verwendung von substituierten Pyrazolinderivaten der Formel (I) zur Bekämpfung von Insekten und Spinnentieren.

**9.** Verfahren zur Herstellung von insektiziden und akariziden Mitteln, dadurch gekennzeichnet, daß man substituierte Pyrazolinderivate der Formel (I) mit Streckmitteln und/oder oberflächenaktiven Mitteln

vermischt.

**Claims**

**1.** Substituted pyrazoline derivatives of the general formula (I)

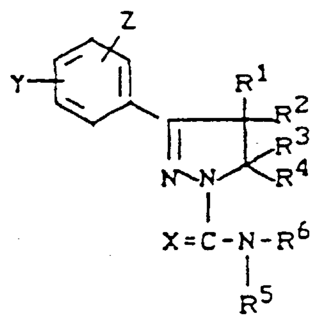

(I)

in which

$R^1$ represents a 1H-pyrrol-1-yl, 1H-pyrazol-1-yl, 1H-imidazol-1-yl, 2H-1,2,3-triazol-2-yl, 1H-1,2,3,-triazol-1-yl, 1H-1,2,4-triazol-1-yl, 4H-1,2,4-triazol-4-yl, 2H-tetrazol-2-yl or 1H-tetrazol-2-yl radical, each of which is optionally substituted by halogen, alkyl($C_1$-$C_6$), CN, $NO_2$, alkoxy($C_1$-$C_6$)-carbonyl, alkyl($C_1$-$C_4$)thio, alkoxy($C_1$-$C_6$), halogenoalkyl($C_1$-$C_4$), halogenoalkyl($C_1$-$C_4$)thio, halogenoalkoxy($C_1$-$C_4$) or dialkyl($C_1$-$C_4$)-amino,

$R^2$ represents hydrogen, alkyl($C_1$-$C_6$), cycloalkyl($C_3$-$C_7$) which is optionally substituted by halogen or halogenoalkyl($C_1$-$C_4$); or halogenoalkyl($C_1$-$C_4$)thio, alkoxy($C_1$-$C_6$)carbonyl or trialkyl-($C_1$-$C_6$)silyl,

$R^3$ represents hydrogen or alkyl($C_1$-$C_6$),

$R^4$ represents hydrogen or alkyl($C_1$-$C_6$),

$R^5$ represents hydrogen, alkyl($C_1$-$C_6$), phenyl or alkyl($C_1$-$C_4$)thio,

$R^6$ represents alkyl($C_1$-$C_6$) which is optionally substituted by halogen, halogenoalkyl($C_1$-$C_4$) or halogenoalkoxy($C_1$-$C_4$),

or represents cycloalkyl($C_3$-$C_7$) which is optionally substituted by halogen, halogenoalkyl($C_1$-$C_4$) or halogenoalkoxy($C_1$-$C_4$), or represents the radical

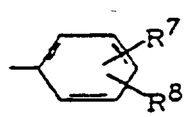

where $R^7$ and $R^8$ can be identical or different and represent hydrogen, halogen, alkyl($C_1$-$C_6$), nitro, cyano, halogenoalkyl($C_1$-$C_6$), alkoxy($C_1$-$C_6$), halogenoalkoxy($C_1$-$C_6$), alkyl($C_1$-$C_6$)thio, halogenoalkyl($C_1$-$C_6$)-thio, phenoxy or phenylthio each of which is optionally substituted by halogen, halogenoalkyl($C_1$-$C_6$), alkoxy($C_1$-$C_6$) or alkyl($C_1$-$C_6$), or represent mono- or dialkylamino each of which has 1 to 6 carbon atoms in the alkyl radical and each of which is optionally substituted by halogen, alkoxy($C_1$-$C_4$) or halogenoalkyl($C_1$-$C_4$), or represent cycloalkyl($C_3$-$C_7$) which is optionally substituted by alkyl($C_1$-$C_6$), alkoxy($C_1$-$C_4$), halogen or alkyl($C_1$-$C_4$)thio, or represent alkoxy($C_1$-$C_6$)carbonyl, alkenyl($C_2$-$C_6$)oxy, alkinyl($C_2$-$C_6$), alkyl-($C_1$-$C_4$)thionyl, alkyl($C_1$-$C_4$)sulphonyl, halogenoalkyl($C_1$-$C_4$)-thionyl or halogenoalkyl($C_1$-$C_4$)-sulphonyl, or where

$R^7$ and $R^8$ together represent one of the following bivalent radicals:

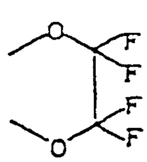 , 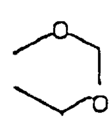 , 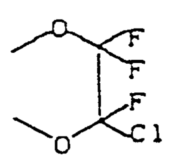 , 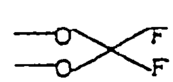 ,

X represents oxygen or sulphur and
Y and Z can be identical or different and represent hydrogen, alkyl($C_1$-$C_6$), halogen, halogenoalkyl($C_1$-$C_6$), alkoxy($C_1$-$C_6$), alkyl($C_1$-$C_6$)thio, halogenoalkoxy($C_1$-$C_4$), halogenoalkyl-($C_1$-$C_4$)thio, alkoxy($C_1$-$C_4$)carbonyl, phenoxy or phenylthio each of which is optionally substituted by halogen, alkyl($C_1$-$C_4$), alkoxy($C_1$-$C_4$) or halogenoalkyl($C_1$-$C_4$), or represent alkenyl-($C_2$-$C_6$)oxy, alkinyl($C_2$-$C_6$), alkyl($C_1$-$C_4$)-thionyl, alkyl($C_1$-$C_4$)sulphonyl, halogenoalkyl($C_1$-$C_4$)-thionyl, halogenoalkyl($C_1$-$C_4$)sulphonyl, mono- or dialkyl($C_1$-$C_6$)amino each of which is optionally substituted by halogen, alkoxy($C_1$-$C_4$) or halogenoalkyl($C_1$-$C_6$), nitro or cyano, or where
Y and Z together represent optionally fluorine-and/or chlorine-substituted 3,4-methylenedioxy or 3,4-ethylenedioxy.

**2.** Substituted pyrazoline derivatives of the formula (I) according to Claim 1, in which

$R^1$ represents a 1H-pyrrol-1-yl, 1H-pyrazol-1-yl, 1H-imidazol-1-yl, 2H-1,2,3,-triazol-2-yl, 1H-1,2,3-triazol-1-yl, 1H-1,2,4-triazol-1-yl, 4H-1,2,4-triazol-4-yl, 2H-tetrazol-2-yl or 1H-tetrazol-1-yl radical, each of which is optionally substituted by fluorine, chlorine, bromine, iodine, alkyl($C_1$-$C_4$), CN, $NO_2$, alkoxy($C_1$-$C_4$)carbonyl, alkyl($C_1$-$C_3$)thio, alkoxy($C_1$-$C_4$), halogenoalkyl($C_1$-$C_3$) halogenoalkyl($C_1$-$C_3$)thio or halogenoalkoxy($C_1$-$C_4$)carbonyl,

$R^2$ represents hydrogen, alkyl($C_1$-$C_4$), optionally fluorine-, chlorine-, bromine- or halogenoalkyl-($C_1$-$C_3$)-substituted cycloalkyl($C_3$-$C_6$), halogenoalkyl($C_1$-$C_3$), halogenoalkyl($C_1$-$C_3$)thio, alkoxy-($C_1$-$C_4$)carbonyl or trialkyl($C_1$-$C_4$)silyl,

$R^3$ represents hydrogen or alkyl($C_1$-$C_4$),

$R^4$ represents hydrogen or alkyl($C_1$-$C_4$),

$R^5$ represents hydrogen, alkyl($C_1$-$C_4$), phenyl or alkyl($C_1$-$C_3$)thio,

$R^6$ represents alkyl($C_1$-$C_4$) which is optionally substituted by fluorine, chlorine, bromine, halogenoalkyl($C_1$-$C_3$) or halogenoalkoxy($C_1$-$C_3$), or represents cycloalkyl($C_3$-$C_6$) which is optionally substituted by fluorine, chlorine, bromine, halogenoalkyl($C_1$-$C_3$) or halogenoalkoxy-($C_1$-$C_3$), or represents the radical

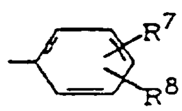

where $R^7$ and $R^8$ can be identical or different and represent
hydrogen, fluorine, chlorine, bromine, iodine, alkyl($C_1$-$C_4$), nitro, cyano, halogenoalkyl($C_1$-$C_3$), alkoxy($C_1$-$C_4$), halogenoalkoxy($C_1$-$C_3$), alkyl($C_1$-$C_3$)thio, halogenoalkyl($C_1$-$C_3$)thio, or phenoxy which is optionally substituted by fluorine, chlorine, bromine, halogenoalkyl($C_1$-$C_3$), alkoxy($C_1$-$C_3$) or alkyl($C_1$-$C_3$), or represent mono- or dialkylamino each of which has 1 to 4 carbon atoms in the alkyl radical and each of which is optionally substituted by fluorine, chlorine, bromine, alkoxy($C_1$-$C_3$) or halogenoalkyl($C_1$-$C_3$), or represent cycloalkyl($C_3$-$C_6$) which is optionally substituted by alkyl($C_1$-$C_3$), alkoxy($C_1$-$C_3$), fluorine, chlorine, bromine or alkyl($C_1$-$C_3$)thio, or where
$R^7$ and $R^8$ together represent one of the following bivalent radicals:

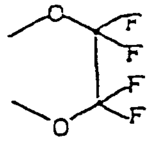 , 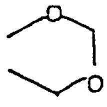 , 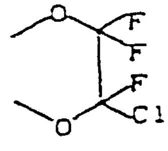 , 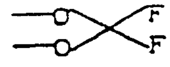 ,

X represents oxygen or sulphur and
Y and Z can be identical or different and represent hydrogen, alkyl($C_1$-$C_4$), fluorine, chlorine, bromine, iodine, halogenoalkyl($C_1$-$C_4$), alkoxy($C_1$-$C_4$), alkyl($C_1$-$C_4$)thio, halogenoalkoxy($C_1$-$C_3$), halogenoalkyl($C_1$-$C_3$)thio, alkoxy($C_1$-$C_3$)carbonyl, phenoxy or phenylthio each of which is optionally substituted by fluorine, chlorine, bromine, alkyl($C_1$-$C_3$), alkoxy($C_1$-$C_3$) or halogenoalkyl($C_1$-$C_3$), or represent halogenoalkoxy($C_1$-$C_3$)carbonyl, or represent alkenyl($C_2$-$C_4$)oxy, alkinyl($C_2$-$C_4$), alkyl($C_1$-$C_3$)thionyl, alkyl($C_1$-$C_3$)sulphonyl, halogenoalkyl($C_1$-$C_3$)thionyl,

halogenoalkyl($C_1$-$C_3$)sulphonyl, mono- or dialkyl($C_1$-$C_3$)amino each of which is optionally substituted by halogen, alkoxy($C_1$-$C_3$) or halogenoalkyl($C_1$-$C_3$), nitro or cyano, or where Y and Z together represent optionally fluorine-and/or chlorine-substituted 3,4-methylenedioxy or 3,4-ethylenedioxy.

**3.** Process for the preparation of substituted pyrazoline derivatives of the general formula (I)

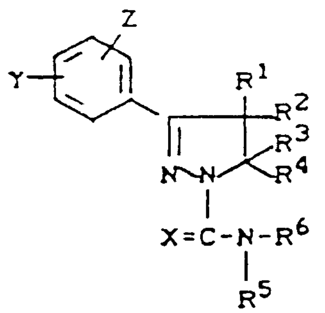

(I)

in which the substituents $R^1$, $R^2$, $R^3$, $R^4$, $R^5$, $R^6$, X, Y and Z have the meaning given in Claim 1, characterized in that

(a) to obtain pyrazoline derivatives of the formula (I) in which $R^5$ represents hydrogen, pyrazoline derivatives of the formula (II)

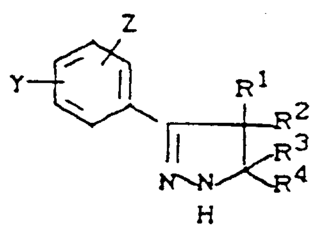

(II)

in which Y, Z, $R^1$, $R^2$, $R^3$ and $R^4$ have the above-mentioned meaning are reacted with isocyanates or isothiocyanates of the formula (III)

$X=C=N\text{-}R^6$ (III)

in which X and $R^6$ have the abovementioned meaning, if appropriate in the presence of bases, or in that

(b) to obtain pyrazoline derivatives of the formula (I) in which $R^2$ represents alkyl($C_1$-$C_6$), cycloalkyl-($C_3$-$C_7$), halogenoalkyl($C_1$-$C_4$)thio, alkoxy($C_1$-$C_6$)carbonyl or trialkyl($C_1$-$C_6$)silyl, compounds of the formula (IV)

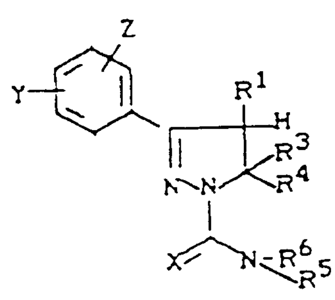

(IV)

are reacted with compounds of the formula (V)

Hal-$R^9$ (V)

in which

Hal represents halogen and

$R^9$ represents alkyl, cycloalkyl, halogenoalkyl, halogenoalkylthio or alkoxycarbonyl,

in an anhydrous medium with the addition of a strong base.

**4.** Substituted pyrazoline derivatives of the general formula (II)

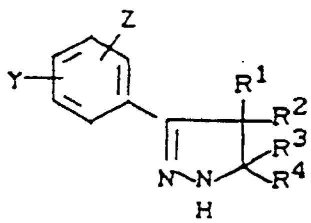

(II)

in which $R^1$, $R^2$, $R^3$, $R^4$, Y and Z have the meaning given in Claim 1.

**5.** Compounds of the general formula (VI)

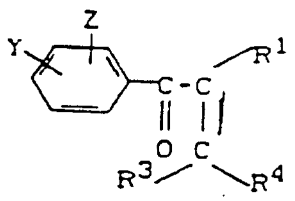

(VI)

in which $R^1$, $R^3$, $R^4$, Y and Z have the meaning given in Claim 1.

**6.** Insecticidal and acaricidal compositions, characterized in that they contain at least one substituted pyrazoline derivative of the formula (I).

**7.** Method of combating insects and arachnids, characterized in that substituted pyrazoline derivatives of the formula (I) are allowed to act on insects and/or arachnids and/or their environment.

**8.** Use of substituted pyrazoline derivatives of the formula (I) for combatting insects and arachnids.

**9.** Process for the preparation of insecticidal and acaricical compositions characterized in that substituted pyrazoline derivatives of the formula (I) are mixed with extenders and/or surface-active agents.

**Revendications**

**1.** Dérivés substitués de pyrazoline, répondant à la formule générale (I)

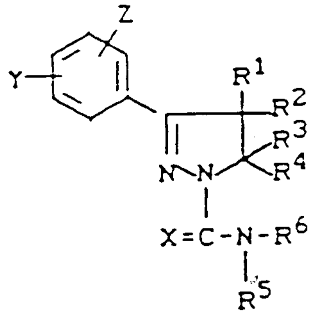

(I)

dans laquelle

$R^1$ représente un radical 1H-pyrrolyle-(1), un radical 1H-pyrazolyle-(1), un radical 1H-imidazolyle-(1), un radical 2H-1,2,3-triazolyle-(2), un radical 1H-1,2,3-triazolyle-(1), un radical 1H-1,2,4-triazolyle-(1), un radical 4H-1,2,4-triazolyle-(4), un radical 2H-tétrazolyle-(2) ou encore un radical 1H-tétrazolyle-(2) éventuellement substitués par un atome d'halogène, par un groupe alkyle en $C_1$-$C_6$, par un groupe CN, par un groupe $NO_2$, par un groupe alcoxy(en $C_1$-$C_6$)-carbonyle, par un groupe alkyl(en $C_1$-$C_4$)thio, par un groupe alcoxy en $C_1$-$C_6$, par un groupe halogénalkyle en $C_1$-$C_4$, par un groupe halogénalkyl(en $C_1$-$C_4$)thio, par un groupe halogénalcoxy en $C_1$-$C_4$, par un groupe dialkyl(en $C_1$-$C_4$)amino,

$R^2$ représente un atome d'hydrogène, un groupe alkyle en $C_1$-$C_6$, un groupe cycloalkyle en $C_3$-$C_7$ éventuellement substitué par un atome d'halogène, par un groupe halogénalkyle en $C_1$-$C_4$; un groupe halogénalkyl(en $C_1$-$C_4$)thio, un groupe alcoxy(en $C_1$-$C_6$)carbonyle ou encore un groupe trialkyl(en $C_1$-$C_6$)silyle,

$R^3$ représente un atome d'hydrogène ou un groupe alkyle en $C_1$-$C_6$,

$R^4$ représente un atome d'hydrogène ou un groupe alkyle en $C_1$-$C_6$,

$R^5$ représente un atome d'hydrogène, un groupe alkyle en $C_1$-$C_6$, un groupe phényle ou un groupe alkyl(en $C_1$-$C_4$)thio,

$R^6$ représente un groupe alkyle en $C_1$-$C_6$ éventuellement substitué par un atome d'halogène, par un groupe halogénalkyle en $C_1$-$C_4$, par un groupe halogénalcoxy en $C_1$-$C_4$; représente un groupe cycloalkyle en $C_3$-$C_7$ éventuellement substitué par un atome d'halogène, par un groupe halogénalkyle en $C_1$-$C_4$, par un groupe halogénalcoxy en $C_1$-$C_4$, ou encore représente le radical

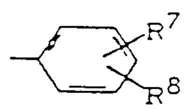

où $R^7$ et $R^8$ peuvent être identiques ou différents et représentent un atome d'hydrogène, un atome d'halogène, un groupe alkyle en $C_1$-$C_6$, un groupe nitro, un groupe cyano, un groupe halogénalkyle en $C_1$-$C_6$, un groupe alcoxy en $C_1$-$C_6$, un groupe halogénalcoxy en $C_1$-$C_6$, un groupe alkyl(en $C_1$-$C_6$)thio, un groupe halogénalkyl(en $C_1$-$C_6$)thio; un groupe phénoxy ou un groupe phénylthio éventuellement substitués par un atome d'halogène, par un groupe halogénalkyle en $C_1$-$C_6$, par un groupe alcoxy en $C_1$-$C_6$, par un groupe alkyle en $C_1$-$C_6$; un groupe mono- ou dialkylamino contenant chacun de 1 à 6 atomes de carbone dans le radical alkyle, éventuellement substitués par un atome d'halogène, par un groupe alcoxy en $C_1$-$C_4$, par un groupe halogénalkyle en $C_1$-$C_4$; un groupe cycloalkyle en $C_3$-$C_7$, un groupe alcoxy(en $C_1$-$C_6$)carbonyle), un groupe alcényl(en $C_2$-$C_6$)oxy, un groupe alcynyle en $C_2$-$C_6$, un groupe alkyl(en $C_1$-$C_4$)thionyle, un groupe alkyl(en $C_1$-$C_4$)sulfonyle, un groupe halogénalkyl(en $C_1$-$C_4$)thionyle, un groupe halogénalkyl(en $C_1$-$C_4$)sulfonyle éventuellement substitués par un groupe alkyle en $C_1$-$C_6$, par un groupe alcoxy en $C_1$-$C_4$, par un atome d'halogène, par un groupe alkyl(en $C_1$-$C_4$)thio, ou bien dans lequel

$R^7$ et $R^8$ représentent ensemble un des radicaux bivalents ci-après :

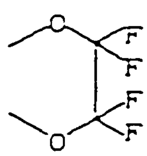 , 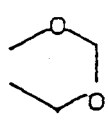 , 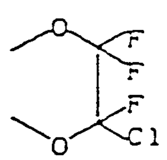 , 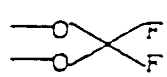 ,

X représente un atome d'oxygène ou un atome de soufre et

Y et Z peuvent être identiques ou différents et représentent un atome d'hydrogène, un groupe alkyle en $C_1$-$C_6$, un atome d'halogène, un groupe halogénalkyle en $C_1$-$C_6$, un groupe alcoxy en $C_1$-$C_6$, un groupe alkyl(en $C_1$-$C_6$)thio, un groupe halogénalcoxy en $C_1$-$C_4$, un groupe halogénalkyl(en $C_1$-$C_4$)thio, un groupe alcoxy(en $C_1$-$C_4$)carbonyle; un groupe phénoxy ou un groupe phénylthio éventuellement substitués par un atome d'halogène, par un groupe

alkyle en $C_1$-$C_4$, par un groupe alcoxy en $C_1$-$C_4$, par un groupe halogénalkyle en $C_1$-$C_4$; un groupe alcényl(en $C_2$-$C_6$)oxy, un groupe alcynyle en $C_2$-$C_6$, un groupe alkyl(en $C_1$-$C_4$)-thionyle, un groupe alkyl(en $C_1$-$C_4$)sulfonyle, un groupe halogénalkyl(en $C_1$-$C_4$)thionyle, un groupe halogénalkyl(en $C_1$-$C_4$)sulfonyle; un groupe nitro, un groupe cyano, un groupe mono- ou dialkyl(en $C_1$-$C_6$)amino éventuellement substitués par un atome d'halogène, par un groupe alcoxy en $C_1$-$C_4$, par un groupe halogénalkyle en $C_1$-$C_4$, ou encore dans lequel
Y et Z représentent ensemble un groupe 3,4-méthylènedioxy ou un groupe 3,4-éthylènedioxy éventuellement substitués par un atome de fluor et/ou par un atome de chlore.

**2.** Dérivés substitués de pyrazoline, répondant à la formule (I) selon la revendication 1, dans laquelle

$R^1$ représente un radical 1H-pyrrolyle-(1), un radical 1H-pyrazolyle-(1), un radical 1H-imidazolyle-(1), un radical 2H-1,2,3-triazolyle-(2), un radical 1H-1,2,3-triazolyle-(1), un radical 1H-1,2,4-triazolyle-(1), un radical 4H-1,2,4-triazolyle-(4), un radical 2H-tétrazolyle-(2) ou un radical 1H-tétrazolyle-(1) éventuellement substitués par un atome de fluor, par un atome de chlore, par un atome de brome, par un atome d'iode, par un groupe alkyle en $C_1$-$C_4$, par un groupe CN, par un groupe $NO_2$, par un groupe alcoxy(en $C_1$-$C_4$)carbonyle, par un groupe alkyl(en $C_1$-$C_3$)thio, par un groupe alcoxy en $C_1$-$C_4$, par un groupe halogénalkyle en $C_1$-$C_3$, par un groupe halogénalkyl(en $C_1$-$C_3$)thio ou encore par un groupe halogénalcoxy(en $C_1$-$C_4$)-carbonyle,

$R^2$ représente un atome d'hydrogène, un groupe alkyle en $C_1$-$C_4$; un groupe cycloalkyle en $C_3$-$C_6$, un groupe halogénalkyle en $C_1$-$C_3$, un groupe halogénalkyl(en $C_1$-$C_3$)thio, un groupe alcoxy(en $C_1$-$C_4$)carbonyle ou un groupe trialkyl(en $C_1$-$C_4$)silyle, éventuellement substitués par un atome de fluor, par un atome de chlore, par un atome de brome, par un groupe halogénalkyle en $C_1$-$C_3$,

$R^3$ représente un atome d'hydrogène ou un groupe alkyle en $C_1$-$C_4$,

$R^4$ représente un atome d'hydrogène ou un groupe alkyle en $C_1$-$C_4$,

$R^5$ représente un atome d'hydrogène, un groupe alkyle en $C_1$-$C_4$, un groupe phényle ou un groupe alkyl(en $C_1$-$C_3$)thio,

$R^6$ représente un groupe alkyle en $C_1$-$C_4$ éventuellement substitué par un atome de fluor, par un atome de chlore, par un atome de brome, par un groupe halogénalkyle en $C_1$-$C_3$, par un groupe halogénalcoxy en $C_1$-$C_3$; un groupe cycloalkyle en $C_3$-$C_6$ éventuellement substitué par un atome de fluor, par un atome de chlore, par un atome de brome, par un groupe halogénalkyle en $C_1$-$C_3$, par un groupe halogénalcoxy en $C_1$-$C_3$, ou encore représente le radical

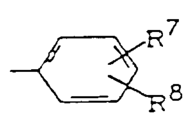

où $R^7$ et $R^8$ peuvent être identiques ou différents et représentent un atome d'hydrogène, un atome de fluor, un atome de chlore, un atome de brome, un atome d'iode, un groupe alkyle en $C_1$-$C_4$, un groupe nitro, un groupe cyano, un groupe halogénalkyle en $C_1$-$C_3$, un groupe alcoxy en $C_1$-$C_4$, un groupe halogénalcoxy en $C_1$-$C_3$, un groupe alkyl(en $C_1$-$C_3$)thio, un groupe halogénalkyl(en $C_1$-$C_3$)thio; un groupe phénoxy éventuellement substitué par un atome de fluor, par un atome de chlore, par un atome de brome, par un groupe halogénalkyle en $C_1$-$C_3$, par un groupe alcoxy en $C_1$-$C_3$, par un groupe alkyle en $C_1$-$C_3$; un groupe mono- ou dialkylamino contenant chacun de 1 à 4 atomes de carbone dans le radical alkyle, éventuellement substitués par un atome de fluor, par un atome de chlore, par un atome de brome, par un groupe alcoxy en $C_1$-$C_3$, par un groupe halogénalkyle en $C_1$-$C_3$; un groupe cycloalkyle en $C_3$-$C_6$ éventuellement substitué par un groupe alkyle en $C_1$-$C_3$, par un groupe alcoxy en $C_1$-$C_3$, par un atome de fluor, par un atome de chlore, par un atome de brome, par un groupe alkyl(en $C_1$-$C_3$)thio, ou encore dans lequel
$R^7$ et $R^8$ représentent ensemble un des radicaux bivalents ci-après :

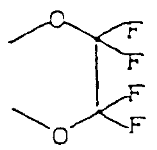 , 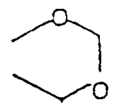 , 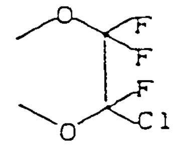 , 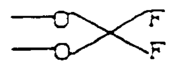 ,

X représente un atome d'oxygène ou un atome de soufre et

Y et Z peuvent être identiques ou différents et représentent un atome d'hydrogène, un groupe alkyle en $C_1$-$C_4$, un atome de fluor, un atome de chlore, un atome de brome, un atome d'iode, un groupe halogénalkyle en $C_1$-$C_4$, un groupe alcoxy en $C_1$-$C_4$, un groupe alkyl(en $C_1$-$C_4$)thio, un groupe halogénalcoxy en $C_1$-$C_3$, un groupe halogénalkyl(en $C_1$-$C_3$)-thio, un groupe alcoxy(en $C_1$-$C_3$)carbonyle; un groupe phénoxy ou un groupe phénylthio éventuellement substitués par un atome de fluor, par un atome de chlore, par un atome de brome, par un groupe alkyle en $C_1$-$C_3$, par un groupe alcoxy en $C_1$-$C_3$, par un groupe halogénalkyle en $C_1$-$C_3$; un groupe halogénalcoxy(en $C_1$-$C_3$)carbonyle, un groupe alcényl(en $C_2$-$C_4$)oxy, un groupe alcynyle en $C_2$-$C_4$, un groupe alkyl(en $C_1$-$C_3$)thionyle, un groupe alkyl-(en $C_1$-$C_3$)sulfonyle, un groupe halogénalkyl(en $C_1$-$C_3$)thionyle, un groupe halogénalkyl(en $C_1$-$C_3$)sulfonyle; un groupe nitro, un groupe cyano, un groupe mono- ou dialkyl(en $C_1$-$C_3$)-amino éventuellement substitués par un atome d'halogène, par un groupe alcoxy en $C_1$-$C_3$, par un groupe halogénalkyle en $C_1$-$C_3$, ou dans lequel

Y et Z représentent ensemble un groupe 3,4-méthylènedioxy ou un groupe 3,4-éthylènedioxy éventuellement substitués par un atome de fluor et/ou par un atome de chlore.

**3.** Procédé pour la préparation de dérivés substitués de pyrazoline, répondant à la formule (I)

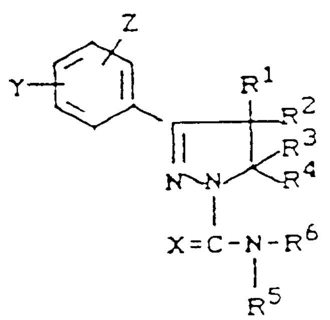

(I)

dans laquelle les substituants $R^1$, $R^2$, $R^3$, $R^4$, $R^5$, $R^6$, X, Y et Z ont la signification indiquée dans la revendication 1,
caractérisé en ce que

(a) pour obtenir les dérivés de pyrazoline de formule (I) dans laquelle $R^5$ représente un atome d'hydrogène, on fait réagir des dérivés de pyrazoline de formule (II)

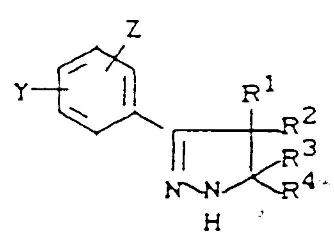

(II)

dans laquelle Y, Z, $R^1$, $R^2$, $R^3$ et $R^4$ ont la signification indiquée ci-dessus,
avec des isocyanates ou des isothiocyanates de formule (III)

$X = C = N\text{-}R^6$ (III)

dans laquelle X et $R^6$ ont la signification indiquée ci-dessus,
éventuellement en présence de bases,
ou bien en ce que
(b) pour obtenir des dérivés de pyrazoline de formule (I) dans laquelle $R^2$ représente un groupe alkyle en $C_1$-$C_6$, un groupe cycloalkyle en $C_3$-$C_7$, un groupe halogénalkyl(en $C_1$-$C_4$)thio, un groupe alcoxy(en $C_1$-$C_6$)carbonyle ou encore un groupe trialkyl(en $C_1$-$C_6$)silyle, on fait réagir des composés de formule (IV)

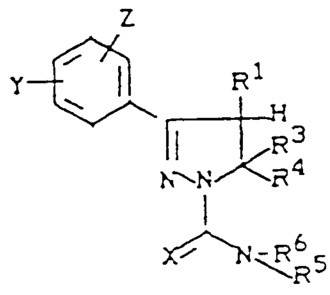

(IV)

avec des composés de formule (V)

Hal-$R^9$ (V)

dans laquelle
Hal représente un atome d'halogène et
$R^9$ représente un groupe alkyle, un groupe cycloalkyle, un groupe halogénalkyle, un groupe halogénalkylthio ou encore un groupe alcoxycarbonyle,
dans un milieu anhydre avec addition d'une base forte.

**4.** Dérivés substitués de pyrazoline selon la formule générale (II)

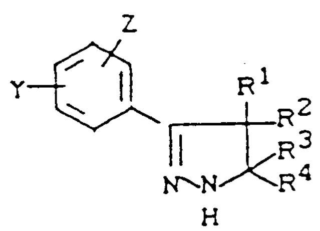

(II)

dans laquelle
$R^1$, $R^2$, $R^3$, $R^4$, Y et Z ont la signification indiquée dans la revendication 1.

**5.** Composés répondant à la formule générale (VI)

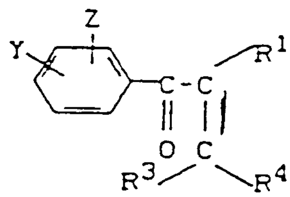

(VI)

dans laquelle
$R^1$, $R^3$, $R^4$, Y et Z ont la signification indiquée dans la revendication 1.

**6.** Agents insecticides et acaricides, caractérisés par une teneur en au moins un dérivé substitué de pyrazoline répondant à la formule (I).

**7.** Procédé pour lutter contre les insectes et les arachnides, caractérisé en ce qu'on laisse agir des dérivés substitués de pyrazoline de formule (I) sur des insectes et/ou sur des arachnides et/ou sur leur biotope.

**8.** Utilisation de dérivés substitués de pyrazoline de formule (I) pour lutter contre les insectes et les arachnides.

**9.** Procédé pour la préparation d'agents insecticides et acaricides, caractérisé en ce qu'on mélange des dérivés substitués de pyrazoline de formule (I) avec des diluants et/ou des agents tensioactifs.